# EUROPEAN PATENT APPLICATION

(11) **EP 4 779 295 A2**
(43) Date of publication of application: **22.07.2026**
(21) Application number: 26168412.0
(22) Date of filing: 02.02.2024
(51) Int. Cl.: G01N 21/64

(54) **SYSTEMS, DEVICES, AND METHODS FOR FLUORESCENCE IMAGING WITH IMAGING PARAMETER MODULATION**

(30) Priority: 02.02.2023 US 202363482892 P
(62) Divisional of application: 24155499.7
(71) Applicant: Moleculight Inc., Toronto ON M5G 1T6 (CA)
(72) Inventor: HIRSON, Desmond, Toronto, Ontario, M5G 1T6 (CA); MORGAN, Garry, Toronto, Ontario, M5G 1T6 (CA); KIRMAN, Jeffrey R., Toronto, Ontario, M5G 1T6 (CA); DACOSTA, Ralph S., Toronto, Ontario, M5G 1T6 (CA); SALEMI, Samuel, Toronto, Ontario, M5G 1T6 (CA)
(74) Representative: PGA S.p.A., Milano, Succursale di Lugano

(57) **Abstract**

Systems, devices, and methods provide for the operation of a portable, hand-held device comprising an illumination device including at least one excitation light source and a driver configured to drive the at least one excitation light source to sequentially produce a plurality of output intensities; an imaging device configured to capture a plurality of fluorescence images of a target surface respectively corresponding to the plurality of output intensities; a memory; and a processor configured to: co-register the plurality of fluorescence images, divide an image area into a plurality of sections, for each of the plurality of sections, select an image portion from one of the plurality of fluorescence images, and combine the selected image portions to generate a composite image.

## Description

### TECHNICAL FIELD

Systems, devices, and methods for fluorescence imaging are disclosed. In particular, the systems, devices, and methods may capture, interpret, and/or modify images using a fluorescence imaging system to identify or assist in the identification of characteristics related to a target. In various applications, for example, the target may be a wound and the systems, devices, and methods herein may be used to identify, quantify, and/or differentiate bacteria present in the wound.

### BACKGROUND

Wound care is a major clinical challenge. Healing and chronic non-healing wounds are associated with a number of biological tissue changes including inflammation, necrosis, production of exudate, bleeding, proliferation, remodeling of connective tissues, and, a common major concern, bacterial presence, growth and infection. A portion of wound infections are not clinically apparent and contribute to the growing personal, emotional, and economic burdens associated with wound care, especially in aging populations. For example, *Pseudomonas aeruginosa* and *Staphylococcus aureus* are genera of bacteria that are prevalent in hospital settings and are common causes of bacterial infection. Currently, the clinical gold standard of wound assessment includes direct visual inspection of the wound site under white light illumination for classical signs and symptoms of infection. This is often combined with a swab culture or tissue biopsy sample for laboratory testing.

Certain medical specialties (*e.g.,* cardiology, oncology, neurology, orthopedics, etc.) rely on particular imaging modalities (*e.g.,* x-ray, ultrasound, magnetic resonance imaging (MRI), computed tomography (CT) scans, etc.) to assist with the diagnosis and assessment. Clinicians in such specialties may use advanced and established methods of interpreting the images. In wound care specialties, by contrast, the standard of care has not historically relied on such imaging modalities and no such advanced or established methods of interpreting the images exist. While some clinicians may use cameras to capture images of a wound in a standard photographic format, these formats do not identify or expose any bacterial information within the wound.

Qualitative and subjective visual assessment only provides a gross view of the wound site, but does not provide information about underlying biological, biochemical, and molecular changes that are occurring at the tissue and cellular level. Moreover, bacteria are invisible to the unaided eye, resulting in suboptimal wound sampling and an inability to appropriately track changes in bacterial growth in the wound site. This can impede healing and timely selection of the optimum anti-microbial treatment. Moreover, it may be difficult to differentiate certain markers of bacterial presence from similar markers caused by non-bacterial sources. For example, a fluorescence image may contain reflections from non-bacterial sources (e.g., tattoos, fingernails, toenails, jewelry, background environment, etc.) which appear to be similar in color to the fluorescence that would be expected from certain strains of bacteria. Moreover, very high or very low bacterial loads may be difficult or impossible to detect with a high degree of confidence using comparative devices due to insufficient signal-to-noise ratios (SNRs) and/or image saturation. These situations may result in the misidentification of a wound as containing bacteria when it does not (perhaps resulting in medically unnecessary treatments) and/or the misidentification of a wound as being free from bacteria when it is in fact infected (potentially delaying treatment).

Therefore, there exists a need for systems, devices, and methods for the capturing of medical images (such as fluorescence images of wound) which may reliably indicate the presence of regions which potentially have bacteria levels above a certain threshold, thus reducing both morbidity and mortality due especially to chronic wounds.

### OVERVIEW

In view of these and other circumstances, the present disclosure provides for methods, systems, and devices to provide point-of-care interpretation of fluorescence images to assist with the identification of regions of bacteria.

In one aspect of the present disclosure, there is provided a portable, hand-held device comprising: an illumination device including at least one excitation light source and a driver configured to drive the at least one excitation light source to sequentially produce a plurality of output intensities; an imaging device configured to capture a plurality of fluorescence images of a target surface respectively corresponding to the plurality of output intensities; a memory; and a processor configured to: co-register the plurality of fluorescence images, divide an image area into a plurality of sections, for each of the plurality of sections, select an image portion from one of the plurality of fluorescence images, and combine the selected image portions to generate a composite image.

In another aspect of the present disclosure, there is provided a system comprising: a display device; an illumination device including an excitation light source and a driver configured to drive the excitation light source to sequentially produce a plurality of output intensities; an imaging device configured to capture a plurality of fluorescence images of a target surface respectively corresponding to the plurality of output intensities; a housing; and circuitry disposed within the housing, the circuitry including a processor configured to: co-register the plurality of fluorescence images, divide an image area into a plurality of sections, for each of the plurality of sections, select an image portion from one of the plurality of fluorescence images, and combine the selected image portions to generate a composite image.

In yet another aspect of the present disclosure, there is provided a fluorescence image interpretation method, comprising: capturing a plurality of fluorescence images of a target surface, including sequentially for a plurality of different values of a drive parameter: driving an excitation light source to produce excitation light at an output intensity corresponding to the drive parameter, and capturing a respective fluorescence image of the target surface, wherein the fluorescence image includes an emission response of the target surface to the excitation light; co-registering the plurality of fluorescence images; dividing an image area into a plurality of sections; for each of the plurality of sections, selecting an image portion from one of the plurality of fluorescence images; and combining the selected image portions to generate a composite image.

In this manner, aspects of the present disclosure provide for improvements in at least the technical field of fluorescence imaging, as well as the related technical fields of image processing, medical devices, biophotonics, wound treatment, and the like. Additional aspects of the present disclosure will be set forth in part in the description which follows, and in part will be obvious from the description or may be learned by practice of the present disclosure. The aspects of the present disclosure, and advantages which arise therefrom, may be realized and attained by means of the elements and combinations particularly pointed out in the appended claims and their equivalents.

It is to be understood that both the foregoing general description and the following detailed description are exemplary and explanatory only and are not restrictive of the present disclosure and claims.

### BRIEF DESCRIPTION OF THE DRAWINGS

These and other aspects of the present disclosure are described with respect to the attached drawings, in which:
FIGS. 1A-B illustrate an exemplary device in accordance with various aspects of the present disclosure;
FIG. 2 illustrates another exemplary device in accordance with various aspects of the present disclosure;
FIG. 3 illustrates an exemplary schematic of a device in accordance with various aspects of the present disclosure;
FIG. 4 illustrates an exemplary image sensor for use with exemplary devices in accordance with various aspects of the present disclosure;
FIG. 5 illustrates an exemplary light source for use with exemplary devices in accordance with various aspects of the present disclosure;
FIG. 6 illustrates an exemplary timing diagram for sequential images in accordance with various aspects of the present disclosure;
FIG. 7 illustrates an exemplary graph of output power level for sequential images in accordance with various aspects of the present disclosure;
FIGS 8-11 illustrate exemplary process flows in accordance with various aspects of the present disclosure; and
FIGS. 12A-13G illustrate exemplary images accordance with various aspects of the present disclosure.

### DETAILED DESCRIPTION

Reference will now be made in detail to various exemplary embodiments, examples of which are illustrated in the accompanying drawings. The various exemplary embodiments are not intended to limit the disclosure. To the contrary, the disclosure is intended to cover alternatives, modifications, and equivalents of the exemplary embodiments. In the drawings and the description, similar elements are provided with similar reference numerals. It is to be noted that the features explained individually in the description can be mutually combined in any technically expedient manner and disclose additional embodiments of the present disclosure.

The present disclosure provides devices, systems, and computer-implemented methods which provide for fluorescence imaging in which a light source and/or an image sensor is modulated to capture a series of fluorescence images respectfully corresponding to different levels of excitation light and/or different exposure periods. The different images may then be mosaicked and/or stitched together to generate a composite image capable of rendering a wide range of component (*e.g.,* bacteria) concentrations in a target surface. While the following description is presented with regard to an exemplary implementation in which the target surface is a wound, devices, systems, and computer-implemented methods in accordance with the present disclosure may be used to detect contamination on non-organic target surfaces in addition to organic tissue surfaces. As such, the target surface may be, without limitation, a wound, a tissue sample, a surgical instrument, a surgical suite, a medical device, forensic equipment, food processing equipment, and the like.

In comparative devices and systems, a fluorescence imaging operation may capture only a single fluorescence image corresponding to a single excitation light source power level and a single exposure period (herein also referred to as a "nominal fluorescence image" or a fluorescence image obtained at a "nominal power level" or a "nominal exposure period"). Such comparative devices and systems may thus be capable of detecting bacterial loads above a certain threshold level. However, if the bacterial load is below this threshold level, the nominal power level and/or exposure period may be insufficient to provide an adequate SNR for a particular area of the target surface thus resulting in a bacterial infection which is not detected. If the bacterial load is sufficiently above this threshold level, the signal may become saturated in the resulting image, thus rendering it difficult or impossible to adequately classify or categorize the source of the signal.

Thus, devices, systems, and computer-implemented methods in accordance with the present disclosure may provide for improved confidence in fluorescence signature interpretation, may result in a reduced occurrence of false negatives and/or false positives, and may be used to provide a qualitative or semi-qualitative estimate of bacterial load.

Although the present disclosure is described primarily with regard to imaging of wound components (e.g., collagen, blood and/or vasculature, oxygenation and/or perfusion, bone, adipose tissue, exudate, and bacteria) and identification of characteristics captured in fluorescent images, the devices, systems, and methods disclosed herein can also be used to capture images and identify characteristics of excised tissue, such as cancerous tissue *(e.g.,* lumpectomy for breast cancer surgery) captured in fluorescent images. In use with excised tissue, the devices and methods could be used to identify characteristics such as, for example, tissue components, tumor size, tumor edge, tumor boundaries, and tissue vascularization shown in fluorescent images. Moreover, while the present disclosure is described primarily with regard to certain wavelengths of fluorescence and corresponding or associated bacterial species, and so on, the present disclosure is not so limited. In practical implementations, the devices, systems, and methods herein may be recalibrated for any bacterial species based on a unique fluorescence emission signature of a given wavelength(s) or wavelength range(s) for the bacterial species, and similarly, can be used with other tissue components (normal or abnormal) for which a unique fluorescence signature is known or for which a signature of a unique biomarker or of a combination of biomarkers contained therein is known. The present disclosure may additionally be implemented with regard to non-bacterial signatures, including but not limited to viruses, fungi, yeast, and/or other microorganisms. The devices, systems, and methods disclosed herein are not limited to use with fluorescence images, and instead may be used to analyze other types of images such as white-light images.

Exemplary wound monitoring devices described herein including include hand-held/portable optical digital imaging devices having specific excitation light sources and optical filters (e.g., low-pass filters, high-pass filters, band-pass filters, multi-band filters, polarization filters, etc.) attached thereto, although in some implementations the hand-held/portable optical digital imaging devices described herein may be filterless. Such exemplary devices may be configured with optical heads to permit multiple different use cases, including but not limited to endoscopic imaging, and in some implementations such devices may be modular. These devices include but are not limited to those described in International Patent Application Publication WO 2009/140757 A1, International Patent Application Publication WO 2019/148268 A1, International Patent Application Publication WO 2020/0148725 A1, and/or International Patent Application Publication WO 2020/0148726 A1, the entire contents of which are herein each incorporated by reference in their entirety. Using imaging devices and systems further described herein, fluorescence of components in a wound due to exposure to excitation light may be imaged and analyzed. For example, in a wound having a bacterial presence caused by or containing, for example, *Pseudomonas aeruginosa,* the *Pseudomonas aeruginosa* fluoresce with a specific spectral signature, *i.e.,* one or more bands of wavelengths with known peaks, when subjected to excitation light. The excitation light may comprise any light with known wavelength or range of wavelengths with known peaks, such as a peak at 405 nm. Capturing and analyzing this data permits identification of bacterial presence in general, and identification of the presence of specific types of bacteria as well. In order to identify, type, and quantify the bacterial presence as well as additional characteristics of the wound, the devices and systems are trained.

One example of a wound monitoring device is a portable, handheld imaging system that includes an imaging device having two or more cameras (i.e., camera sensors) and a processor coupled to the imaging device for analyzing the images captured from the camera sensors to perform algorithms or other operations as will be described in more detail below. The imaging device, for example, includes a first, primary camera sensor and a second, secondary camera sensor. The first, primary camera sensor and the second, secondary camera sensor may be configured to capture standard, white light (WL) images, fluorescent (FL) images, near infrared (NIR) images, or infrared (IR) images. The sensors may be so configured by use with dedicated filters or filters selectable from a plurality of filters associated with the imaging device (e.g., filter wheel, tunable filters, etc.), in which the filters may be wavelength filters (e.g., low-pass filters, high-pass filters, band-pass filters, multi-band filters, etc.) and/or polarization filters. Thus, the method disclosed herein may be used to measure features captured in WL, FL, NIR, or IR images. In some implementations, to permit determination of the parallax value of a primary and secondary image (taken, respectively, by the primary and secondary camera sensors), the first camera sensor is separated from the second camera sensor by a predetermined, fixed separation distance. In other implementations, to permit depth measurement, a time-of-flight or other depth imaging sensor may be provided. In some implementations, the imaging device may include a thermal image sensor to capture thermal images.

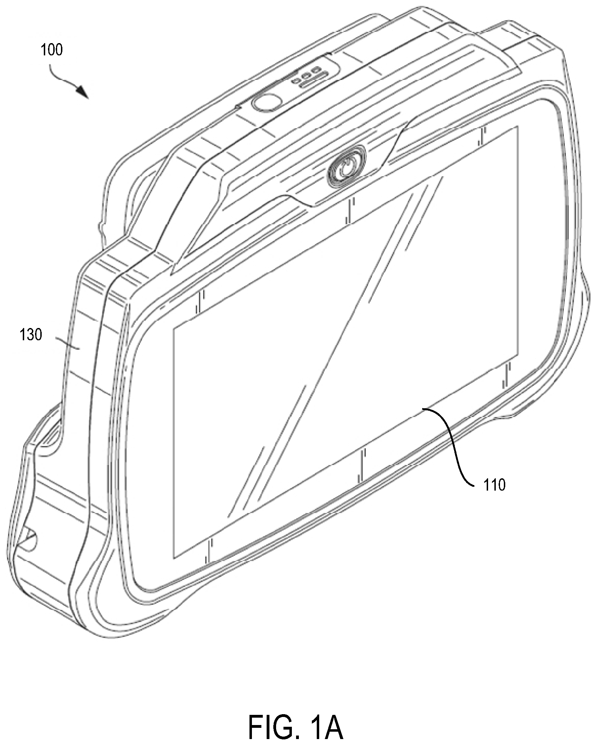

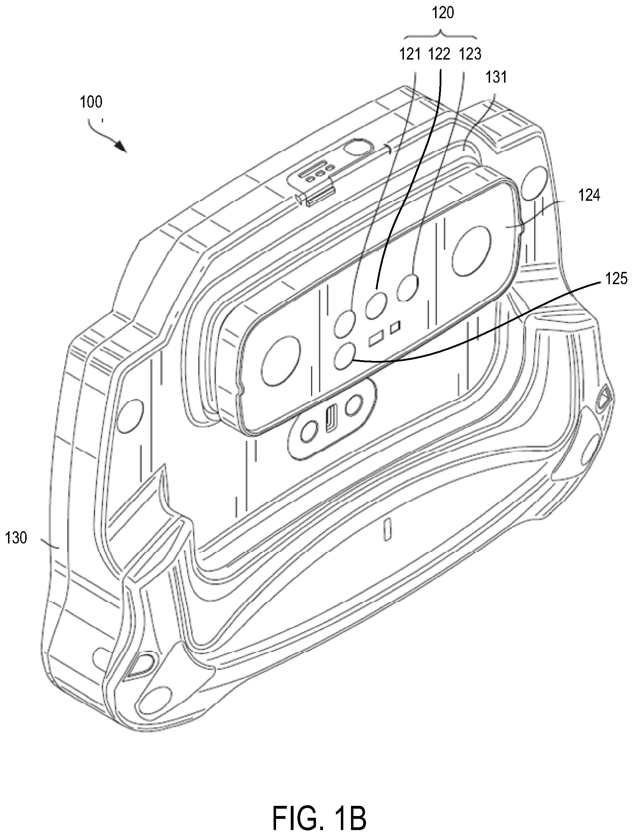

FIGS. 1A-B illustrate an exemplary wound monitoring device 100 in accordance with the present disclosure, in which FIG. 1A is a front perspective view of the wound monitoring device 100 and FIG. 1B is a rear perspective view of the wound monitoring device 100. Device 100 can be, for instance, the MolecuLight DX^{®} device developed by MolecuLight^{®}. Device 100 is non-contact and no imaging contrast agents are required for white light and/or fluorescence imaging. As illustrated, the wound monitoring device 100 includes a display device 110, and imaging device 120, and a housing 130.

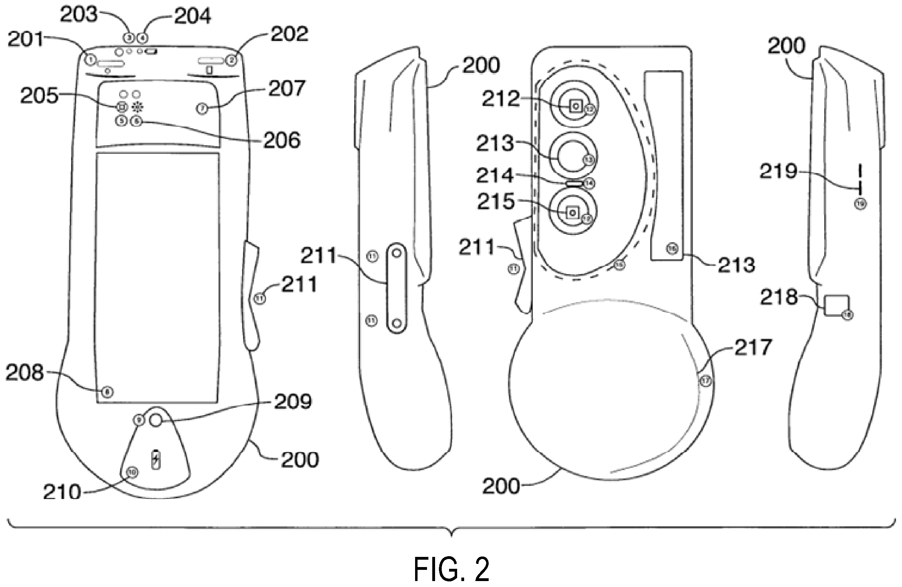

The imaging device 120 includes at least one image sensor, such as, for example, a first image sensor 121, a second image sensor 122, and a third image sensor 123. Each of the first image sensor 121, the second image sensor 122, and the third image sensor 123 may individually be implemented as image sensors that may be used for one or more of WL, FL, IR, and thermal imaging. In one example, the first image sensor 121 and the third image sensor 123 are together configured for stereoscopic white-light imaging, and the second image sensor 122 is configured for fluorescence imaging. In another example, the first image sensor 121 and the third image sensor 123 are together configured for stereoscopic fluorescence imaging, and the second image sensor 122 is configured for white-light imaging. In yet another example, the first image sensor 121 is configured for white-light imaging, the second image sensor 122 is configured for fluorescence imaging of a first wavelength or wavelength range, and the third image sensor 123 is configured for fluorescence imaging of a second wavelength or wavelength range. The physical arrangement *(i.e.,* ordering) of the first image sensor 121, the second image sensor 122, and the third image sensor 123 may also be different from that shown in FIG. 1B. Although for ease of illustration the wound monitoring device 100 depicts three image sensors, the present disclosure includes an imaging device 120 including any number of image sensors so long as at least one image sensor capable of receiving fluorescence signals is present. In the illustration of FIG. 2, the first image sensor 121, the second image sensor 122, and the third image sensor 123 are arranged in an optical battery 124. The optical battery 124 may further include one or more excitation light sources, such as excitation light source 125 implemented as a light-emitting diode (LED). The optical battery 124 may additionally include at least one white light torch, rangefinder, temperature sensor, etc.

The housing 130 may include a physical user interface, such as a power button, one or more input buttons, and so on. The housing 130 may also include various input and output ports, such as wired charging ports, inductive charging ports, universal serial bus (USB) ports, and/or other peripheral ports. As shown in FIG. 1B, the housing 130 includes an optical mount 131 on which the imaging device 120 is mounted. The optical mount 131 may provide for a permanent physical mount or may provide for a removable mount in implementations where the imaging device 120 is modular *(i.e.,* may be replaced with other imaging devices such as endoscope heads). The housing 130 may be of a unitary construction or may include multiple housing components attached together. While not particularly illustrated in FIGS. 1A-B, the housing 130 may include an internal space in which various components such as a processor *(e.g.,* a central processing unit (CPU)), a memory, a program storage, and the like are disposed.

FIG. 2 illustrates four views (front, right side, rear, and left side elevational views) of another exemplary wound monitoring device 200 in accordance with the present disclosure. Device 200 can be, for instance, the MolecuLight i:X^{®} device developed by MolecuLight^{®}. Device 200 allows clinicians to quickly, safely, and easily visualize bacterial presence and distribution in skin and wounds, in real-time including but not limited to the point-of-care. Device 200 is non-contact and no imaging contrast agents are required for white light and/or fluorescence imaging. Device 200 is depicted as a handheld portable medical device comprised of a high-resolution color LCD display and touch-sensitive screen 208 with integrated optical and microelectronic components and internal battery power source. Device 200 further includes a power button 201 for turning the device on and off, a display screen power button 202 for turning display screen 208 on and off, a system status LED 203 indicating overall device performance, a battery status LED 204 indicating device battery charge, a range finder LED system 205 indicating an optimal distance from the wound being targeted or imaged, an ambient light status LED 206 for indicating an optimal lighting environment for fluorescence mode imaging, a heat sink 207 for dissipating heat as device 200 may get warm after prolonged use, a home button 209 for providing access to image and video capture functions of device 200, and a port 210 for charging and data transfer. Port 210 may be used with any universal or proprietary cable, such as USB, or a MolecuLight i:X^{®} connecting cable.

Device 200 further includes a rocker switch 211 enabling switching between a standard imaging mode and a fluorescence imaging mode. For instance, device 200 captures real-time images *(e.g.,* in JPG format), and videos *(e.g.,* in MOV format) using both standard and fluorescent imaging modes. The standard imaging mode is generally used for standard photography, i.e., to capture RGB images and videos of targets illuminated with standard white light. The fluorescence imaging mode is used to capture RGB images and videos of targets illuminated with light having known peak wavelengths and intended to generate fluorescence from specific targets being excited by the light. Consequently, device 200 further includes LEDs 212 that have specific wavelengths or ranges of wavelengths for illuminating targets when in fluorescence imaging mode, as well as a camera lens 213 enabling image and video capture, a range finder sensor 214 for detecting an optimal distance from a wound or surrounding skin, and an ambient light sensor 215 for detecting optimal lighting conditions for the fluorescence imaging mode. Further, device 200 includes a holding contour 217 for allowing a user to grip the device securely, and a charging port 218 enabling device charging using a standard or proprietary power adapter.

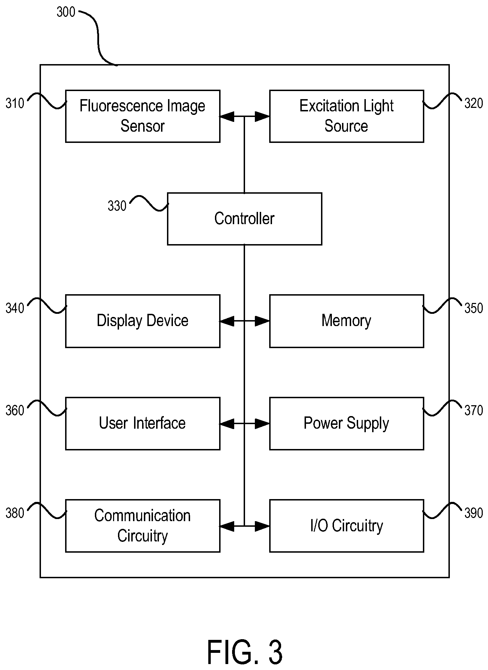

FIG. 3 illustrates an exemplary schematic of a wound monitoring device 300 in accordance with the present disclosure, which may be the same as or similar to the devices 100 or 200 illustrated in FIGS. 1-2, or a different device. As illustrated in FIG. 3, the wound monitoring device 300 includes a fluorescence image sensor 310, an excitation light source 320, a controller 330, a display device 340, a memory 350, a user interface (UI) 360, a power supply 370, communication circuitry 380, and input/output (I/O) circuitry 390.

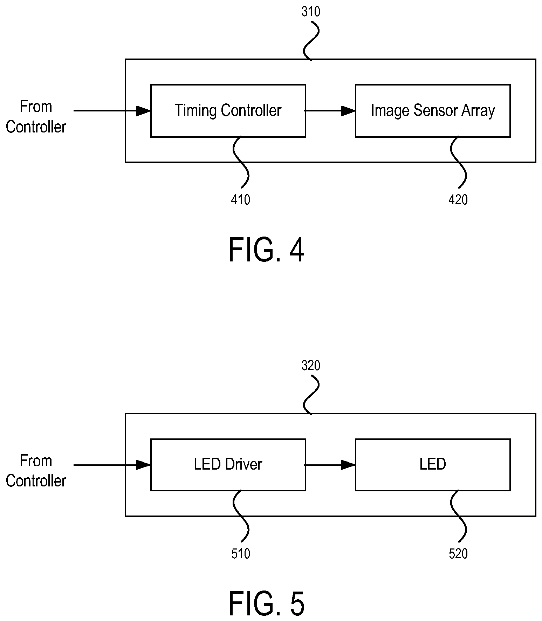

The fluorescence image sensor 310 is configured to capture one or a plurality of fluorescence images of a target surface *(e.g.,* a wound), and may be implemented as or including a photoelectric conversion device which receives incident electromagnetic radiation *(e.g.,* light) and converts the radiation into signal charges which may be used to generate an image of the field-of-view of the image sensor. Such an image sensor may have, for example, a complementary metal-oxide semiconductor (CMOS) architecture, a charge-coupled device (CCD) architecture, and so on. The photoelectric conversion device may include an array of a plurality of individual pixel circuits, each of which includes a photosensitive element such as a photodiode. The image sensor may include additional circuitry such as driving circuits, timing circuits, memory circuits, control circuits, output circuits, power circuits, buses, and the like. For example, the fluorescence image sensor 310 may include a timing control circuit configured to modulate an exposure period for images captured by the fluorescence image sensor 310. One example of a fluorescence image sensor 310 which includes a timing control circuit is illustrated in FIG. 4.

As illustrated in FIG. 4, the fluorescence image sensor 310 includes a timing controller 410 and an image sensor array 420. The image sensor array 420 may be a two-dimensional array of photosensitive pixel circuits, such as CMOS pixel circuits. The timing controller 410 is configured to receive a control signal from the controller 330 and translate the control signal into a corresponding timing signal. The image sensor array 420 is configured to capture images in accordance with the timing signal, such that the timing signal controls the exposure period (*i.e.,* the length of time to which the photosensitive elements of the pixel circuits accumulate charge and/or are exposed to light). By changing the timing signal, the timing controller may be configured to drive the image sensor array 420 to produce a plurality of output images, each corresponding to a different exposure period, also referred to as "modulating" the image sensor array 420.

Returning to FIG. 3, the fluorescence image sensor 310 may include a filter or a plurality of filters to block certain wavelengths and/or polarizations of light while permitting other wavelengths and/or polarizations of light to pass therethrough to the fluorescence image sensor 310. In implementations where a plurality of filters is present, the filters may be disposed on a device which allows individual filters to be individually selected and moved in front of the fluorescence image sensor 310 (*e.g.,* a filter wheel) to selectively detect specific optical signals based on the wavelength of light. The fluorescence image sensor 310 may be part of a digital camera, for example having at least an ISO800 sensitivity, but more preferably an ISO3200 sensitivity, and may be combined with one or more optical emission filters, or other equally effective (*e.g.,* miniaturized) mechanized spectral filtering mechanisms (*e.g.,* acousto-optical tunable filter or liquid crystal tunable filter). Each optical filter may have different discrete spectral bandwidths and may be band-pass, multi-band, short-pass, or long-pass filters. In one example, the fluorescence image sensor 310 includes a first filter having a passband of 600-660 nm and/or a second filter having a passband of 470-520 nm. In such an example, the fluorescence image sensor may be said to be configured detect wavelengths of 600-600 nm and/or 470-520 nm, although in practice the fluorescence image sensor 310 may be configured to detect all wavelengths of visible light incident thereon and, through filtering, only wavelengths of a certain range may in fact be incident on the fluorescence image sensor 310. The preceding wavelength ranges are exemplary and not exhaustive, and in practical implementations filters having additional, fewer, and/or different passbands may be provided. Moreover, in implementations where no filter is provided, the fluorescence image sensor 310 may be configured to detect all visible wavelengths and/or a portion of wavelengths in the IR and/or UV ranges. The fluorescence image sensor 310 may include a lens, which may be aligned to point at a target area (*e.g.,* an area being imaged), and can detect the optical signal that emanates from the target area or surface.

The excitation light source 320 may be or include one or more light emitting elements that produce excitation light or illumination. The excitation light or illumination may be broadband (*e.g.,* 300-1200 nm) or may be narrowband or multi-narrowband (*e.g.,* one or a combination of 300-350 nm, 325-375 nm, 350-400 nm, 375-425 nm, 400-450 nm, 425-475 nm, 450-500 nm, 475-525 nm, 500-550 nm, 525-575 nm, 550-600 nm, 575-625 nm, 600-650 nm, 625-675 nm, 650-700 nm, 725-775 nm, 700-750 nm, 725-775 nm, 750-800 nm, 775-825 nm, 800-850 nm, 825-875 nm, 850-900 nm, 875-925 nm, 900-950 nm, 925-975 nm, 950-1000 nm, 975-1025 nm, 1000-1050 nm, 1025-1075 nm, 1050-1100 nm, 1075-1125 nm, 1100-1150 nm, 1125-1175 nm, or 1150-1200 nm), for example, monochromatic or white light having a wavelength peak of 400-450 nm, or any other combination of single or multiple wavelengths (*e.g.,* wavelengths in the ultraviolet/visible/near infrared/infrared ranges), to illuminate a target object *(e.g.,* a wound or other area of interest) in order to elicit an optical signal (*e.g.,* fluorescence). For example, the excitation light source 320 may be blue or violet LED arrays emitting light at about 405 nm (e.g., ± 5 nm, ± 10 nm, ± 15 nm, or ± 20 nm), and may be coupled with additional band-pass filters centered at about 405 nm to remove/minimize the side spectral bands of light from the LED array output so as not to cause light leakage into the imaging detector with its own optical filters. It should be understood that the foregoing wavelengths are merely exemplary and not exhaustive. In some implementations, the narrowband or multi-narrowband wavelengths may have any peak wavelength between 300 nm and 1200 nm and are not limited to a bandwidth of 50 nm. For example, the above list of wavelengths may include wavelengths within 15 nm of the above values, such that "about 400-450 nm" includes 385-400 nm, 415-400 nm, 400-465 nm, 403-444 nm, and so on. The excitation light source 320 may further or alternatively comprise a laser diode and/or filtered lights arranged in a variety of geometries. The device 300 may include a method or apparatus (e.g., a heatsink or a cooling fan) to dissipate heat and cool the excitation light source 320. The device 300 may include a system or device (e.g., an optical band-pass filter) to remove any undesirable wavelengths of light from the excitation light source 320 used to illuminate the object being imaged. The excitation light source 320 may include a light source driver operatively connected to the light emitting elements included in the excitation light source 320. The light source driver may, for example, receive a control signal (such as from the controller 330) and generate a corresponding drive signal which causes the light emitting elements to produce excitation light or illumination of a corresponding intensity, pattern, duration, etc. One example of an excitation light source 320 which includes a light source driver and light emitting elements is illustrated in FIG. 5.

As illustrated in FIG. 5, the excitation light source 320 includes an LED driver 510 and an LED 520. The LED 520 may be a standard LED, an organic LED, or a quantum-dot LED. The present disclosure is not limited solely to LED architecture, but instead may implement a laser architecture (*e.g.,* with a laser driver and a laser light source). The LED driver 510 is configured to receive a control signal from the controller 330 and translate the control signal into a corresponding drive signal. The LED 520 is configured to emit excitation light in accordance with the drive signal. In some implementations, the drive signal is a current signal and the LED 520 emits light with an intensity that is proportional to an amplitude of the drive signal. In other implementations, the drive signal is a pulse-width modulation (PWM) signal and the LED 520 emits light with an intensity that is proportional to the duty cycle of the drive signal (*i.e.,* to the ratio of ON time to OFF time within a period of the drive signal) or is a voltage signal and the LED 520 emits light with an intensity that is proportional to the voltage differential across the LED 520. By changing the drive signal, the LED driver 510 may be configured to drive the LED 520 to sequentially produce a plurality of output intensities, also referred to as "modulating" the LED 520. For purposes of explanation, the present disclosure describes the drive signal as a current signal, such that the LED 520 produces an output power in accordance with the amplitude of the drive signal.

Returning to FIG. 3, the controller 330 may be an electronic processor, such as one or more computer processors (processing units), microprocessors, digital signal processors, controllers and microcontrollers, etc. Logic may be formed from signals stored on a computer-readable medium such as memory 350 that, in an exemplary embodiment, may be a random access memory (RAM), read-only memories (ROM), erasable/electrically erasable programmable read-only memories (EPROMS/EEPROMS), flash memories, etc. Logic may also comprise digital and/or analog hardware circuits, for example, hardware circuits comprising logical AND, OR, XOR, NAND, NOR, and other logical operations. Logic may be formed from combinations of software and hardware. On a network, logic may be programmed on a server, or a complex of servers. A particular logic unit is not limited to a single logical location on the network. Moreover, the modules need not be executed in any specific order. Each module may call another module when needed to be executed. The controller 330 may include a trained machine learning algorithm configured to perform one or more of the operations described in more detail below.

The display device 340 may be any type of display, including but not limited to a liquid crystal display (LCD), a quantum dot display, an organic light emitting display (OLED), a thin-film transistor (TFT) display, and the like. The display device 340 may be configured to provide real-time display of the field-of-view of the fluorescence image sensor 310, to display images stored in memory 350 or remotely stored, to provide graphical overlays, and so on. The display device 340 may include a touch panel to permit input from a clinician as a user interface. The display device 340 may be configured to present one or more graphical user interfaces (GUIs).

The UI 360 may include physical and virtual mechanisms by which a user interacts with the device 300, including physical buttons, dials, switches, and the like; interactive icons displayed on the display device 340; and/or one or more GUIs. The power supply 370 may be an AC/DC power supply, a compact battery bank, or a rechargeable battery pack. Additionally or alternatively, the device 300 may be adapted for connecting to an external power supply. The communication circuitry 380 may include wired communication circuitry and/or wireless communication circuitry to permit communications between the device 300 and external devices. The communication circuitry 380 may be configured to communicate using wired communication media such as coaxial cables, USB cables, Ethernet cables, and so on; and/or using wireless communication protocols such as Wi-Fi, Bluetooth, Near Field Communication (NFC), 4G cellular communications, 5G wireless communications, and so on.

The I/O circuitry 390 may be or include on or more of: an interface for a head-mounted display; an interface for an external printer; an interface for a tablet computer, laptop computer, desk top computer or other computer device; an interface for a device allowing the use of extra memory; and an interface for a microphone. The device 300 may have a housing that houses all the components in one entity. The housing may be equipped with a means of securing any digital imaging device within it. The housing may be designed to be hand-held, compact, and/or portable. The housing may be one or more enclosures or separate housings.

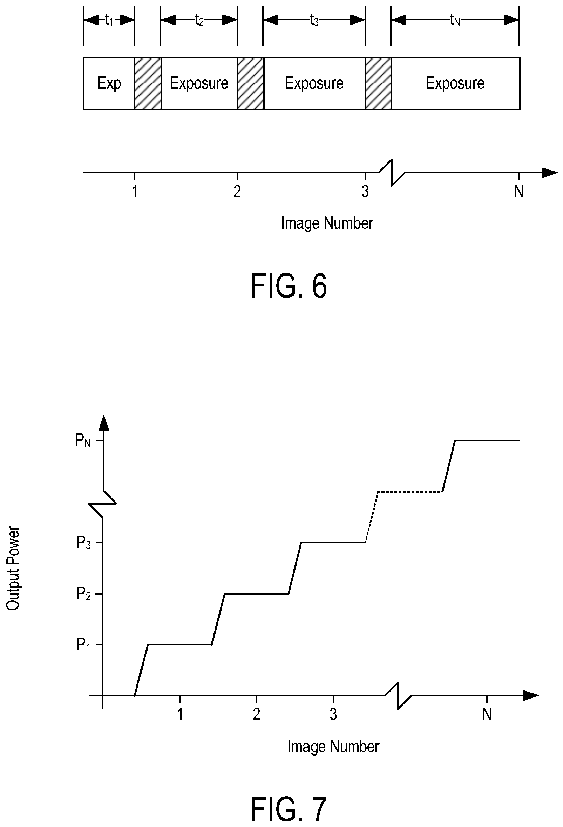

The device 300 may be configured with application programs (*e.g.,* stored in the memory 350 and loaded by the controller 330 for execution) to perform various algorithms, including those to provide excitation light source modulation and/or fluorescence image sensor modulation for fluorescence images, image processing, image interpretation, and so on. FIG. 6 illustrates one exemplary method by which the controller 330 *(e.g.,* using the timing controller 410) modulates the fluorescence image sensor 310 so that the fluorescence image sensor 310 captures a plurality of different fluorescence images at different exposure periods, whereas FIG. 7 illustrates one exemplary method by which the controller 330 *(e.g.,* using the LED driver 510) modulates the excitation light source 320 so that the fluorescence image sensor 310 captures a plurality of different fluorescence images at different output power levels.

In FIG. 6, the horizontal axis generally corresponds to time. The modulation illustrated in FIG. 6 may be performed automatically in response to a predetermined trigger, such as user input via the UI 360. In FIG. 6, the wound monitoring device 300 captures *N* sequential images, with each image *i* corresponding to imaging at a different exposure period *tᵢ.* For purposes of illustration, FIG. 6 also shows an interstitial period between successive exposures, during which various blanking and reset operations may be performed so as to output and/or store one image and prepare the pixel circuit for capture of the next image. The value of *N* is a positive integer greater than one and may be user selectable. In some implementations, one, several, or all of the exposure periods *tᵢ* may be user selectable. In other implementations, the exposure periods *tᵢ* may be automatically determined based on a minimum exposure period *t₁* and the total number of images *N.*

In response to the predetermined trigger, the fluorescence image sensor 310 is set to the first exposure period *t₁* and a first fluorescence image is captured. Then, the fluorescence image sensor 310 is set to the second exposure period *t₂* and a second fluorescence image is captured. This repeats until the *N*^{th} fluorescence image is taken at the maximum exposure period *t_{N}.* In this manner, the "maximum" exposure period *t_{N}* does not necessarily mean the maximum exposure period of which the fluorescence image sensor is capable, but instead refers to the longest exposure period for the fluorescence imaging operation. After the *N*^{th} fluorescence image is captured, the exposure period may be reset and a white light image may be captured. While FIG. 6 illustrates the exposure periods as successively lengthening, in practical implementations the exposure periods may follow any order.

In one particular example, *N* = *4, tᵢ =* 25 milliseconds (ms), *t₂ =* 75 ms, *t₃ =* 100 ms, and *t₄ =* 200 ms. In this example, when a user initiates a fluorescence imaging operation *(e.g.,* by pressing a button on the UI 360 or by entering a command via a GUI presented on the display device 340), the wound monitoring device 300 captures four imaging devices over ** seconds. The fluorescence imaging may be automatically followed by a white light imaging operation as noted above, as well as by various imaging processing operations as will be described in more detail below.

In FIG. 7, the horizontal axis generally corresponds to time (measured in terms of an index number of successive captured images) and the vertical axis corresponds to the output power level of the excitation light source 320. The modulation illustrated in FIG. 7 may be performed automatically in response to a predetermined trigger, such as a user input via the UI 360. In some implementations, the sequential imaging takes place at a predetermined rate, such as two images per second; however, in other implementations the frame rate (and by extension exposure period) may vary such that the predetermined rate may range from 5 images per second to 100 images per second and/or may be user selectable. In FIG. 7, the wound monitoring device 300 captures *N* sequential images, with each image *i* corresponding to excitation light at a different output power level *Pᵢ.* The value of *N* is a positive integer greater than 1, and may be user selectable. In some implementations, one, several, or all of the output power levels *Pᵢ* may be user selectable. In other implementations, the output power levels *Pᵢ* may be automatically determined based on a maximum output power level *P_{N}* and the total number of images *N.* The output power levels *Pᵢ* may be regularly spaced (such that the difference between *Pᵢ₋₁* and *Pᵢ* is equal to the difference between *Pᵢ and Pᵢ₊₁* for all *i*) or irregularly spaced.

In response to the predetermined trigger, the excitation light source 320 is driven to the first output power level *P₁* and a first fluorescence image is captured. Then, the excitation light source 320 is driven to the second output power level *P₂* and a second fluorescence image is captured. This repeats until the excitation light source 320 is driven to the maximum output power level *P_{N}* and the *N*^{th} fluorescence image is taken. In this manner, the "maximum" output power level *P_{N}* does not necessarily mean the maximum output power level of which the excitation light source 320 is capable, but instead refers to the highest output power for the fluorescence imaging operation. After the *N*^{th} fluorescence image is captured, driving of the excitation light source 320 stops such that the output power level returns to zero. At this point (or, in other implementations, prior to capture of the first fluorescence image), a white light image may be captured.

FIG. 5 illustrates the drive signal as a monotonically-increasing stepped waveform, such that each successive fluorescence image corresponds to a higher output power level. In other implementations, the drive signal may be a monotonically-decreasing stepped waveform beginning with the maximum output power level *P_{N}* for the first fluorescence image and ending with the lowest output power level *P₁* for the *N*^{th} fluorescence image. The stepped waveform need not, however, be monotonic and the output power levels may be presented in any order desired. In still other implementations, the drive signal may have a pulsed waveform in which the excitation light source 320 is driven to the first output power level *P₁* for capture of the first fluorescence image, driving is subsequently stopped such that the drive signal returns to zero, then the excitation light source 320 is driven to the second output power level *P₂* for capture of the second fluorescence image, driving is subsequently stopped such that the drive signal returns to zero, and so on. Of course, if the drive signal has a pulsed waveform the pulses may be arranged in order increasing amplitude from *P₁* to *P_{N},* in order of decreasing amplitude from *P_{N}* to *P₁,* or in any other order. In yet other implementations, the drive signal may have a ramp waveform which steadily increases or decreases. In such implementations, the output power level would not be constant throughout the entire exposure period for image capture and thus the output power level may refer to the average power level for the given image capture period.

In one particular example *N = 4, P₁* = 0.44 milliwatts (mW), *P₂ =* 2.3 mW, *P₃* = 4.2 mW, and *P₄* = 6.2 mW. In another particular example *N =* 6, *P₁* = 0.44 milliwatts (mW), *P₂ =* 1.44 mW, *P₃* = 2.45 mW, *P₄* = 3.46 mW, *P₅* = 4.5 mW, and *P₆* = 5.5 mW. In these examples, when a user initiates a fluorescence imaging operation (*e.g.,* by pressing a button on the UI 360 or by entering a command via a GUI presented on the display device 340), the wound monitoring device 300 captures four or six fluorescence images over two or three seconds, respectively. The fluorescence imaging operation may be automatically followed by a white light imaging operation as noted above, as well as by various image processing operations as will be described in more detail below.

In some implementations, LED modulation and exposure period modulation may be used together. However, it is also contemplated that only LED modulation or only exposure period modulation may be utilized. Moreover, a combination of LED and exposure period modulation may be used, for example to capture a first set of images at a first output power level for *n₁* different exposure periods, followed by a second set of images at a second power level for *n₂* different exposure periods, and so on. In still other implementations, the output power level of an LED may be iteratively increased to the maximum level *P_{N}* (holding the exposure period constant), after which the exposure period may be iteratively increased to capture additional images at the maximum power level *P_{N}.* In yet other implementations, LED modulation, exposure, period modulation, or both may be implemented in a successive manner with different power levels and/or exposure periods in order to provide coarse modulation followed by fine modulation. For example, after a first iteration of LED modulation from power level *P₁* to *P₅,* a second iteration of LED modulation may be performed from power level *P₁'* to *P₅',* where each of *P₁'* to *P₅'* are between *P₃* and *P₄.*

Returning to FIG. 3, the controller 330 may be configured to "process" a fluorescence image or images by co-registering images, dividing an image area into a plurality of sections or tiles, selecting a best image for each corresponding tile, mosaicking and/or stitching image sections together, and so on. The controller 330 may be configured to "interpret" a fluorescent image (including a composite image) by analyzing, for example, the hue, saturation, and value of colors present in the fluorescent image and identifying, based on the analysis, areas of the image that correspond to one or more areas of bacterial presence, where the bacterial presence exceeds a certain quantitative threshold or a specific type of bacteria that may be based on a spectral signature. The analysis may comprise, for example, comparing color space parameters (*e.g.,* Hue, Saturation, and Value channels in an HSV color space, etc.) of the image to threshold parameters, where the thresholds are set to remove pixels that may or may not be suspicious or contain a threshold quantity of bacteria. The controller 330 may then create an overlay placed on the original image or on another generated image to highlight these areas of the original image and indicate that a clinician should consider these areas suspicious (e.g., as containing a certain level of bacteria and/or a certain type of bacteria) or consider for physical follow up such as debridement or swabbing.

The process of co-registering images may include various operations to determine the spatial relationship between the pixels of one fluorescence image and the pixels of another fluorescence image. For example, the controller 330 may be configured to determine that the upper-left pixel (0,0) of the first fluorescence image corresponds to a different pixel (x,y) of the second fluorescence image. The pixel correspondence may be determined for multiple pixels in the first and second fluorescence images, such that the controller 330 may be able to determine whether and to what degree the wound monitoring device 300 was moved *(i.e.,* translated, rotated about the imaging axis, rotated about other perpendicular axes, and so on) between the capture of the first fluorescence image and the capture of the second fluorescence image. The co-registration operations may be performed for each fluorescence image. Afterward, the controller 330 may be configured to identify a rectangular area of the target surface that is present in all fluorescence images. This rectangular area may be identified as an "image area" for later processing. In some implementations, the process of co-registering images includes a white light image and/or a thermal image in addition to the fluorescence images.

The process of dividing the image may include various operations to section each fluorescence image. For example, the controller 330 may be configured to divide the image area for each fluorescence image into a plurality of tiles. The tiles may be regularly sized and shaped *(e.g.,* a plurality of rectangles of equal size) or irregularly sized and shaped *(e.g.,* polygonal tessellation across the image area). If the tiles are irregularly sized and shaped, the tiles may be larger in areas that are comparatively featureless in the fluorescence images (*e.g.,* portions of the image area where there is little signal and/or where the signal changes little) and smaller in areas that include features (*e.g.,* small areas of brightness or areas where the signal changes quickly from pixel to pixel). In some implementations, the white light image may be used to determine which portions of the image area should have larger tiles and which portions of the image area should have smaller tiles. In other implementations, one or more fluorescence images may additionally or alternatively be used. Regardless of whether the tiles are regular or irregular, the tiling pattern is the same for the image area of each fluorescence image.

The process of selecting a best area may include various operations to determine, on a tile by tile basis, which fluorescence image conveys the best information on the status of the target area. For example, the controller 330 may be configured, for a given tile, to determine the fluorescence signal present in each fluorescence image. The fluorescence signal may be analyzed in terms of either the absolute signal or the SNR of the fluorescence image. Using the SNR instead of the absolute signal may permit the controller 330 to account for instances where the signal is saturated. The fluorescence image having the best value of the fluorescence signal *(e.g.,* the highest SNR) may be flagged or otherwise identified as the best image for the given tile.

The process of mosaicking may include various operations to generate an output image consisting of components selected from multiple different fluorescence images. For example, the controller 330 may be configured to generate a blank image having a dimension corresponding to the image area and having a tiling pattern corresponding to the tiling pattern used for the fluorescence images. For each tile, the controller 330 may be configured to use the image data corresponding to the fluorescence image identified as the best image. The output of this process may be referred to as a "mosaicked image."

The process of stitching may include various operations to smooth or otherwise remove discontinuities present in the mosaicked image. For example, the image data selected for one tile may and the image data selected for an adjacent tile may originate from different source fluorescence images. In such instances, discontinuities may exist at the border between the neighboring tiles where one or more parameters (*e.g.,* one or more of the Hue, Saturation, and Value channels in the HSV color space) abruptly jump from one pixel to the next. To remove such discontinuities, the controller 330 may be configured to modify the image data such that the discontinuous parameters for pixels in the vicinity of the border (*e.g.,* within ten pixels, within fifty pixels, and so on) smoothly transition from one end of the vicinity to the other. The output of this process may be referred to as a "stitched image."

The process of interpreting may include various operations to automatically analyze a processed image (*e.g.,* the mosaicked image and/or the stitched image) to determine the presence and/or concentration of one or more components of the target surface. For example, the processor 330 may be configured to perform a color analysis and/or a textural analysis of the processed image. In one particular example, the color analysis includes converting the processed image to an HSV color space (if it is not already in said color space) to generate a first converted image, comparing a saturation parameter of the first converted image to a first saturation threshold condition, wherein the first saturation threshold condition is based on a correlation between the first color and a first bacterial fluorescence signature, comparing a value parameter of the first converted image to a first value threshold condition, wherein the first value threshold condition is based on the correlation between the first color and the first bacterial fluorescence signature, and flagging areas of the processed image where the saturation parameter satisfies the first saturation threshold condition and the first value parameter satisfies the value threshold condition; unflag the flagged areas which are smaller than a size threshold, and outline the remaining flagged areas on the processed image, thereby to generate an overlay image. The color analysis may be repeated for multiple colors, each having their own saturation threshold conditions and/or value threshold conditions based on the correlations between the individual color and different bacterial fluorescence signatures. In other examples of the present disclosure, a color space other than HSV may be used in the color analysis, including but not limited to HSL, CIEXYZ, CIELUV, L*a*b*, RGB, YCbCr, YUV, LCh, CMY, CMYK, and custom color spaces. Where color spaces other than HSV are used, the threshold conditions (*e.g.,* the hue thresholds, the saturation thresholds, and/or the value thresholds) may be converted to the other color spaces directly. Where the color space used in the color analysis is the same as the color space in which the input image has been captures (*e.g.,* both RGB), then the conversion operation may be omitted.

In a particular example, the textural analysis includes converting the processed image to grayscale to generate a second converted image; comparing, on a pixel-by-pixel basis, an intensity parameter of the second converted image to an intensity threshold; temporarily flagging regions within a predetermined pixel distance of pixels in which the intensity parameter exceeds the intensity threshold; converting the processed image to a L*a*b* color space (if it is not already in said color space), thereby to generate a third converted image; within each channel of the third converted image, determine a respective gradient of the temporarily flagged regions; and permanently flagging those ones of the temporarily flagged regions in which the respective gradient for each channel of the third converted image exceeds a gradient threshold. In other examples of the present disclosure, a color space other than L*a*b* may be used in the textural analysis, including but not limited to HSV, CIEXYZ, CIELUV, RGB, YCbCr, YUV, LCh, CMY, CMYK, and custom color spaces. Where color spaces other than L*a*b* are used, the thresholds (*e.g.,* the intensity thresholds and/or the gradient thresholds) may be selected and/or generated anew based on the characteristics of the other color spaces. Where color spaces other than HSV are used, the threshold conditions (*e.g.,* the hue thresholds, the saturation thresholds, and/or the value thresholds) may be converted to the other color spaces directly. Where the color space used in the textural analysis is the same as the color space in which the input image has been captures *(e.g.,* both RGB), then the conversion operation may be omitted. Where the color space used in the textural analysis is the same as the color space used in the color analysis, then one of the conversion operations may be omitted and/or the two conversion operations may be combined (depending on the order in which the color analysis and the textural analysis occur and/or whether the analyses are conducted in series or parallel). An "overlay image" may refer to a combination of an overlay with the processed image itself, in which the overlay includes contours drawn as a result of the color and/or textural analysis. Alternatively, an "overlay image" may refer to the overlay itself as an element that is shown, stored, etc. separate from the processed image itself, such that no modifications are made to the processed image.

The textural analysis may be used to differentiate between bacterial sources of apparent fluorescence and non-bacterial sources of apparent fluorescence (*e.g.,* bone, tendon, tattoo ink, and so on). The individual bacterial signature may correspond to a bacterial concentration depending on the output power level and/or exposure period at which the corresponding fluorescence image was captured (*e.g.,* 10² colony forming units per gram (cfu/g) or higher if the bacteria was detected only at high output power levels and/or long exposure periods, 10⁷ cfu/g or higher if the bacteria was detected even at low output power levels and/or short exposure periods, 10⁴ cfu/g or higher if the bacteria was first detected at intermediate output power levels and/or medium exposure periods) and may indicate the presence of any one of the following: gram negative aerobic species, including *Pseudomonas aeruginosa, Escherichia coli, Proteus mirabilis, Proteus vulgaris, Enterobacter cloacae, Serratia marcescens, Acinetobacter baumannii, Klebsiella pneumoniae, Klebsiella oxytoca, Morganella morganii, Stenotrophomonas maltophilia, Citrobacter koseri, Citrobacter freundii, Aeromonas hydrophilia, Alcaligenes faecalis,* and *Pseudomonas putida;* gram positive aerobic species, including *Staphylococcus aureus, Staphylococcus epidermis, Staphylococcus lugdunensis, Staphylococcus capitis, Corynebacterium striatum, Bacillus cereus,* and *Listeria monocytogenes;* and/or anaerobic species, including *Bacteroides fragilis*, *Clostridium perfringens*, *Peptostreptococcus anaerobius, Propionibacterium acnes, and Veillonella parvula.* This list is intended to be exemplary only and does not encompass all species to which the identification and differentiation operations may be applied.

The operations of determining a concentration of the component may result in a qualitative or semi-qualitative estimate of the concentration. For example, the concentration may categorize an estimated bacterial load for one or more identified bacterial species as either none, occasional, light, moderate, or heavy. The estimate may be based on which fluorescence image was earlier identified as the best image for areas determined to correspond to the bacterial species. For example, if a particular area of bacterial contamination corresponds to an image area where the best fluorescent image was taken at a low output power level of the excitation light source 320 or a short exposure period of the fluorescence image sensor 310, it may be determined that the bacterial load is heavy. In contrast, if a particular area of bacterial contamination corresponds to an image area where the best fluorescent image was taken at a high output power level of the excitation light source 320 or a long exposure period of the fluorescence image sensor 310, it may be determined that the bacterial load is occasional or light.

Depending on the particular implementation *(i.e.,* depending on whether the stitching and/or overlay processes are formed), any one or more selected from the mosaicked image, the stitched image, the overlay image, or combinations thereof may be output and/or stored as a "composite image," for example on the display device 340 and/or in the memory 350. The composite image may also include indicators, such as labels, contours, arrows, or other interface elements which operate to identify an area of the target surface which meets a predetermined condition, such as an area corresponding to the concentration of various components in the target area. These interface elements may be directly overlaid on the composite image and/or may be included in a border area. The composite image may also include metadata identify the source fluorescence image for each of the plurality of tiles.

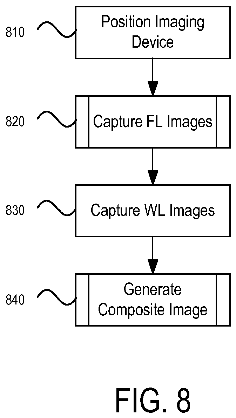

FIGS. 8-11 illustrates one example of operations in accordance with the present disclosure. The operations of FIGS. 8-11 may be performed by a user of a wound monitoring device, such as the exemplary wound monitoring devices 100, 200, and/or 300 described above with regard to FIGS. 1-3.

At operation 810, the user positions the wound monitoring device relative to a target surface, such as a wound or other portion of the skin surface of a patient. The positioning may be accomplished with the assistance of a range finder included in the wound monitoring device; for example, a GUI on a display of the wound monitoring device may be configured to provide a visual indication to the user regarding whether the wound monitoring device is positioned at an appropriate distance from the target surface for imaging.

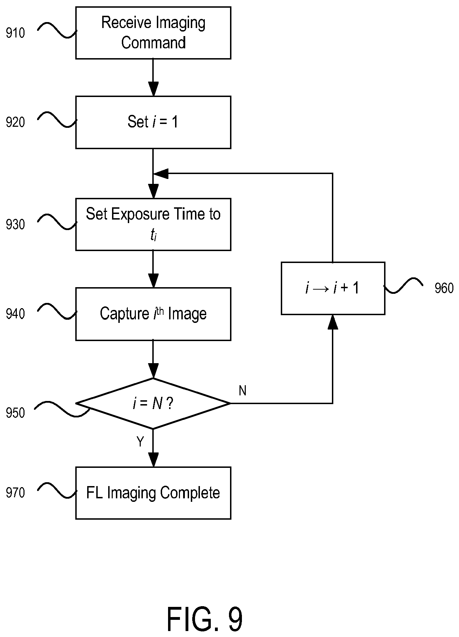

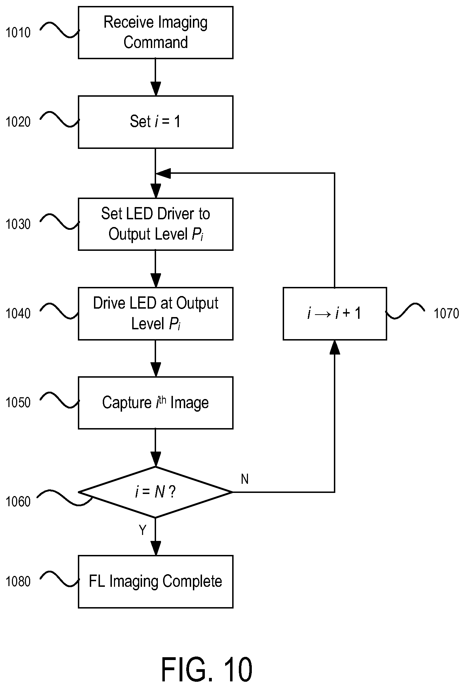

Once the wound monitoring device is in position (*e.g.,* held appropriately by the user), the user may initiate operation 820 to sequentially capture a plurality of images. Operation 820 includes a series of sub-operations, examples of which are illustrated in FIGS. 9 and 10. The user may initiate operation 820 by performing a predetermined trigger operation, such as a user input via a button or switch included on a housing of the wound monitoring device and/or via a command entered via the GUI on the display of the wound monitoring device. Thus, at operation 910 or 1010 (depending on whether exposure period modulation or LED modulation is being performed), the wound monitoring device receives a command to perform fluorescence imaging. The fluorescence imaging includes the capture of *N* sequential images, with each image *i* corresponding to excitation light at a different exposure period *tᵢ* or a different output power level *Pᵢ.* The value of *N* is a positive integer greater than 1, and may be user selectable. In some implementations, one, several, or all of the exposure periods *tᵢ* or the output power levels *Pᵢ* may be user selectable. In other implementations, the exposure periods *tᵢ* or the output power levels *Pᵢ* may be automatically determined based on a minimum exposure period *t₁* or a maximum output power level *P_{N}* and the total number of images *N*.

In response to the imaging command, at operation 920 or 1020 the wound monitoring device initializes an index variable *i* to 1. In the case of exposure period modulation, operation 930 is performed. At operation 930 a timing controller of the fluorescence image sensor controls an image sensor array of the fluorescence image sensor, such that the fluorescence image sensor is configured to capture images at with an exposure period *tᵢ.* In the case of LED modulation, operations 1030 and 1040 are performed. At operation 1030 a light source driver (*e.g.,* an LED driver) of the wound monitoring device sets its output power level to the corresponding output power level *Pᵢ.* At operation 1040, the light source driver drives an excitation light source *(e.g.,* an LED) of the wound monitoring device to produce excitation light at an intensity corresponding to the output power level *Pᵢ.* In either case, synchronously with the emission by the excitation light source, at operation 940 or 1050 the fluorescence image sensor of the wound monitoring device captures a fluorescence image.

At operation 950 or 1060, it is determined whether the fluorescence imaging procedure has completed, for example by determining if the index *i = N.* If the fluorescence imaging procedure is not completed, at operation 960 or 1070 the index i is incremented and the method returns to operation 930 or 1030. Operations 930-940 or 1030-1050 are repeated for the exposure period *tᵢ* or output power level *Pᵢ* corresponding to the new value of the index i. If at operation 950 or 1060 it is determined that the fluorescence imaging procedure is complete, the fluorescence imaging subroutine exits at operation 970 or 1080. In the case of LED modulation, at operation 1080 and the driving of the excitation light source stops such that the output power level returns to zero.

To perform the repeated iterations of operations 1030, 1040, and 1050 *(i.e.,* in the case of LED modulation), the light source driver may provide a drive signal in the form of a monotonically-increasing stepped waveform, such that each successive fluorescence image corresponds to a higher output power level. In other implementations, the drive signal may be a monotonically-decreasing stepped waveform beginning with the maximum output power level *P_{N}* for the first fluorescence image and ending with the lowest output power level *P₁* for the *N*^{th} fluorescence image. The stepped waveform need not, however, be monotonic and the output power levels may be presented in any order desired. In still other implementations, the drive signal may have a pulsed waveform in which the excitation light source is driven to the first output power level *P₁* for capture of the first fluorescence image, driving is subsequently stopped such that the drive signal returns to zero, then the excitation light source is driven to the second output power level *P₂* for capture of the second fluorescence image, driving is subsequently stopped such that the drive signal returns to zero, and so on. Of course, if the drive signal has a pulsed waveform the pulses may be arranged in order increasing amplitude from *P₁* to *P_{N},* in order of decreasing amplitude from *P_{N}* to *P₁,* or in any other order. In yet other implementations, the drive signal may have a ramp waveform which steadily increases or decreases. In such implementations, the output power level would not be constant throughout the entire exposure period for image capture and thus the output power level may refer to the average power level for the given image capture period.

After operation 820 has been completed, (*e.g.,* after all operations of FIGS. 9 or 10 have been completed), a white light image is captured of the target surface at operation 830. Preferably, operation 830 occurs in the absence of any output from the excitation light source and with a nominal exposure period. In some implementations, a white light source (*e.g.,* a white LED) of the wound monitoring device may be illuminated for operation 830. In other implementations, operation 830 may occur under ambient lighting conditions.

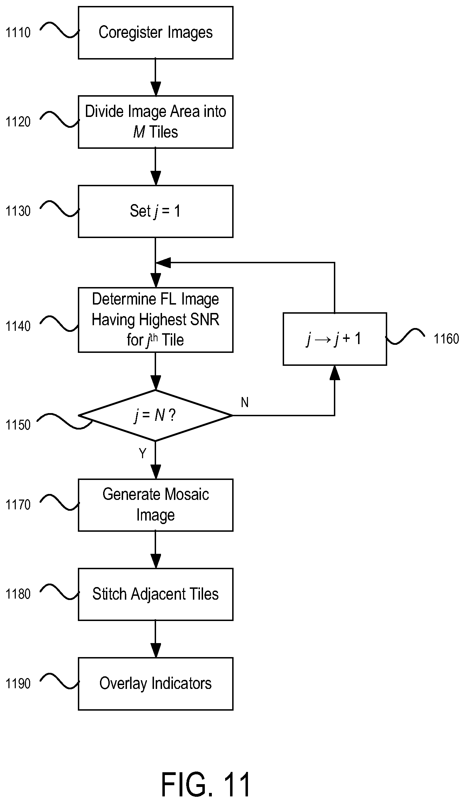

Once all fluorescence images and the white light image have been captured, at operation 840 a composite image is generated. Operation 840 includes a series of sub-operations, an example of which is illustrated in FIG. 11. The operations of FIG. 11 may be performed automatically upon completion of image capture, or may be performed at a later time *(e.g.,* in response to a user input and/or at a predetermined scheduled time).

At operation 1110, the fluorescent images are coregistered. Operation 1110 may include various operations to determine the spatial relationship between the pixels of one fluorescence image and the pixels of another fluorescence image. For example, the wound monitoring device may be configured to determine that the upper-left pixel (0,0) of the first fluorescence image corresponds to a different pixel (x,y) of the second fluorescence image. The pixel correspondence may be determined for multiple pixels in the first and second fluorescence images, such that the wound monitoring device may be able to determine whether and to what degree the wound monitoring device was moved *(i.e.,* translated, rotated about the imaging axis, rotated about other perpendicular axes, and so on) between the capture of the first fluorescence image and the capture of the second fluorescence image. The co-registration operations may be performed for each fluorescence image. Afterward, the wound monitoring device may be configured to identify a rectangular area of the target surface that is present in all fluorescence images. This rectangular area may be identified as an "image area" for later processing. In some implementations, the process of co-registering images includes the white light image and/or a thermal image in addition to the fluorescence images.

At operation 1120, the image area is divided into *M* tiles, where *M* is a positive integer greater than 1. The tiles may be regularly sized and shaped *(e.g.,* a plurality of rectangles of equal size) or irregularly sized and shaped (*e.g.,* polygonal tessellation across the image area). If the tiles are irregularly sized and shaped, the tiles may be larger in areas that are comparatively featureless in the fluorescence images (*e.g.,* portions of the image area where there is little signal and/or where the signal changes little) and smaller in areas that include features (*e.g.,* small areas of brightness or areas where the signal changes quickly from pixel to pixel). In some implementations, the white light image may be used to determine which portions of the image area should have larger tiles and which portions of the image area should have smaller tiles. In other implementations, one or more fluorescence images may additionally or alternatively be used. Regardless of whether the tiles are regular or irregular, the tiling pattern is the same for the image area of each fluorescence image.

For each tile, it is determined which fluorescence image provides the best representation of the target area and/or conveys the best information on the status of the target area. This is represented by a series of operations 1130 to 1160 which are performed for each tile *j,* where *j* is an index variable running from 1 to *M.* At operation 1130 the wound monitoring device initializes the index variable *j* to 1. Then, at operation 1140, the wound monitoring device may determine which fluorescence image should be selected for tile *j.* This may include an operation of determining the fluorescence signal present in each fluorescence image. The fluorescence signal may be analyzed in terms of either the absolute signal or the SNR of the fluorescence image. Using the SNR instead of the absolute signal may permit the method to account for instances where the signal is saturated. The fluorescence image having the best value of the fluorescence signal *(e.g.,* the highest SNR) may be flagged or otherwise identified as the best image for the given tile.

At operation 1150, it is determined whether a fluorescence image has been selected for each tile, for example by determining if the index *j* = *M.* If there remain any tiles which have not yet been processed, at operation 1160 the index *j* is incremented and the method returns to operation 1140. Operations 1140 and 1150 are then repeated for the tile represented by the new value of the index *j.* If at operation 1150 it is determined that all tiles have been processed, at operation 1170 a mosaic image is generated.

Operation 1170 may include various operations to generate an output image consisting of components selected from multiple different fluorescence images. For example, operation 1170 may include generating a blank image having a dimension corresponding to the image area and having a tiling pattern corresponding to the tiling pattern used for the fluorescence images. For each tile, the method may be configured to use the image data corresponding to the fluorescence image identified as the best image. The output of this process may be referred to as a "mosaicked image."

In some instances, the image data selected for one tile may and the image data selected for an adjacent tile may originate from different source fluorescence images. In such instances, discontinuities may exist at the border between the neighboring tiles where one or more parameters (*e.g.,* one or more of the Hue, Saturation, and Value channels in the HSV color space) abruptly jump from one pixel to the next. Therefore, the mosaicked image may be processed according to operation 1180 to stitch the adjacent tiles together. Operation 1180 may include various operations to smooth or otherwise remove discontinuities present in the mosaicked image. To remove such discontinuities, the image data may be modified such that the discontinuous parameters for pixels in the vicinity of the border (*e.g.,* within ten pixels, within fifty pixels, and so on) smoothly transition from one end of the vicinity to the other. The output of this process may be referred to as a "stitched image."

Where desired, the mosaicked image and/or the stitched image may be further processed to provide for automated image interpretation and/or the generation and overlay of various indicators at operation 1190. Operation 1190 may include various operations to automatically analyze a processed image (*e.g.,* the mosaicked image and/or the stitched image) to determine the presence and/or concentration of one or more components of the target surface. For example, the method may include performing a color analysis and/or a textural analysis of the processed image. In one particular example, the color analysis includes converting the processed image to an HSV color space (if it is not already in said color space) to generate a first converted image, comparing a saturation parameter of the first converted image to a first saturation threshold condition, wherein the first saturation threshold condition is based on a correlation between the first color and a first bacterial fluorescence signature, comparing a value parameter of the first converted image to a first value threshold condition, wherein the first value threshold condition is based on the correlation between the first color and the first bacterial fluorescence signature, and flagging areas of the processed image where the saturation parameter satisfies the first saturation threshold condition and the first value parameter satisfies the value threshold condition; unflag the flagged areas which are smaller than a size threshold, and outline the remaining flagged areas on the processed image, thereby to generate an overlay image. The color analysis may be repeated for multiple colors, each having their own saturation threshold conditions and/or value threshold conditions based on the correlations between the individual color and different bacterial fluorescence signatures. In other examples of the present disclosure, a color space other than HSV may be used in the color analysis, including but not limited to HSL, CIEXYZ, CIELUV, L*a*b*, RGB, YCbCr, YUV, LCh, CMY, CMYK, and custom color spaces. Where color spaces other than HSV are used, the threshold conditions *(e.g.,* the hue thresholds, the saturation thresholds, and/or the value thresholds) may be converted to the other color spaces directly. Where the color space used in the color analysis is the same as the color space in which the input image has been captures (*e.g.,* both RGB), then the conversion operation may be omitted.

In a particular example, the textural analysis includes converting the processed image to grayscale to generate a second converted image; comparing, on a pixel-by-pixel basis, an intensity parameter of the second converted image to an intensity threshold; temporarily flagging regions within a predetermined pixel distance of pixels in which the intensity parameter exceeds the intensity threshold; converting the processed image to a L*a*b* color space (if it is not already in said color space), thereby to generate a third converted image; within each channel of the third converted image, determine a respective gradient of the temporarily flagged regions; and permanently flagging those ones of the temporarily flagged regions in which the respective gradient for each channel of the third converted image exceeds a gradient threshold. In other examples of the present disclosure, a color space other than L*a*b* may be used in the textural analysis, including but not limited to HSV, CIEXYZ, CIELUV, RGB, YcbCr, YUV, LCh, CMY, CMYK, and custom color spaces. Where color spaces other than L*a*b* are used, the thresholds (*e.g.,* the intensity thresholds and/or the gradient thresholds) may be selected and/or generated anew based on the characteristics of the other color spaces. Where color spaces other than HSV are used, the threshold conditions (*e.g.,* the hue thresholds, the saturation thresholds, and/or the value thresholds) may be converted to the other color spaces directly. Where the color space used in the textural analysis is the same as the color space in which the input image has been captures *(e.g.,* both RGB), then the conversion operation may be omitted. Where the color space used in the textural analysis is the same as the color space used in the color analysis, then one of the conversion operations may be omitted and/or the two conversion operations may be combined (depending on the order in which the color analysis and the textural analysis occur and/or whether the analyses are conducted in series or parallel). An "overlay image" may refer to a combination of an overlay with the processed image itself, in which the overlay includes contours drawn as a result of the color and/or textural analysis. Alternatively, an "overlay image" may refer to the overlay itself as an element that is shown, stored, etc. separate from the processed image itself, such that no modifications are made to the processed image.

The textural analysis may be used to differentiate between bacterial sources of apparent fluorescence and non-bacterial sources of apparent fluorescence (*e.g.,* bone, tendon, tattoo ink, and so on). The individual bacterial signature may correspond to a bacterial concentration depending on the output power level at which the corresponding fluorescence image was captured (*e.g.,* 10² cfu/g or higher if the bacteria was detected only at high output power levels, 10⁷ cfu/g or higher if the bacteria was detected even at low output power levels, 10⁴ cfu/g or higher if the bacteria was first detected at intermediate output power levels) and may indicate the presence of any one of the following: gram negative aerobic species, including *Pseudomonas aeruginosa, Escherichia coli, Proteus mirabilis, Proteus vulgaris, Enterobacter cloacae, Serratia marcescens, Acinetobacter baumannii, Klebsiella pneumoniae, Klebsiella oxytoca, Morganella morganii, Stenotrophomonas maltophilia, Citrobacter koseri, Citrobacter freundii, Aeromonas hydrophilia, Alcaligenes faecalis,* and *Pseudomonas putida;* gram positive aerobic species, including *Staphylococcus aureus, Staphylococcus epidermis, Staphylococcus lugdunensis, Staphylococcus capitis, Corynebacterium striatum, Bacillus cereus, and Listeria monocytogenes;* and/or anaerobic species, including *Bacteroides fragilis*, *Clostridium perfringens*, *Peptostreptococcus anaerobius, Propionibacterium acnes,* and *Veillonella parvula.* This list is intended to be exemplary only and does not encompass all species to which the identification and differentiation operations may be applied.

Operation 890 may include the generation of a qualitative or semi-qualitative estimate of the concentration. For example, the concentration may categorize an estimated bacterial load for one or more identified bacterial species as either none, occasional, light, moderate, or heavy. The estimate may be based on which fluorescence image was earlier identified as the best image for areas determined to correspond to the bacterial species. For example, if a particular area of bacterial contamination corresponds to an image area where the best fluorescent image was taken at a low output power level of the excitation light source, it may be determined that the bacterial load is heavy. In contrast, if a particular area of bacterial contamination corresponds to an image area where the best fluorescent image was taken at a high output power level of the excitation light source, it may be determined that the bacterial load is occasional or light.

Depending on the particular implementation *(i.e.,* depending on whether the stitching and/or overlay processes are formed), any one or more selected from the mosaicked image, the stitched image, the overlay image, or combinations thereof may be output and/or stored as a "composite image," for example on the display of the wound monitoring device and/or in a memory of the wound monitoring device. The composite image may also include indicators, such as labels, contours, arrows, or other interface elements which operate to identify an area of the target surface which meets a predetermined condition, such as an area corresponding to the concentration of various components in the target area. These interface elements may be directly overlaid on the composite image and/or may be included in a border area. The composite image may also include metadata identify the source fluorescence image for each of the plurality of tiles

The operations of FIGS. 8-11 may be performed in a different order than the order expressly shown in FIGS. 8-11. For example, in some implementations the white light image capture of operation 830 may be performed before the fluorescence image capture procedure of operation 820 occurs. Moreover, in accordance with the present disclosure, certain operations of FIGS. 8-11 may be omitted. For example, if there are no (or only minimal) discontinuities present in the mosaicked image, operation 1180 may be omitted. Moreover, where there is no need to provide image interpretation, operation 1190 may be omitted.

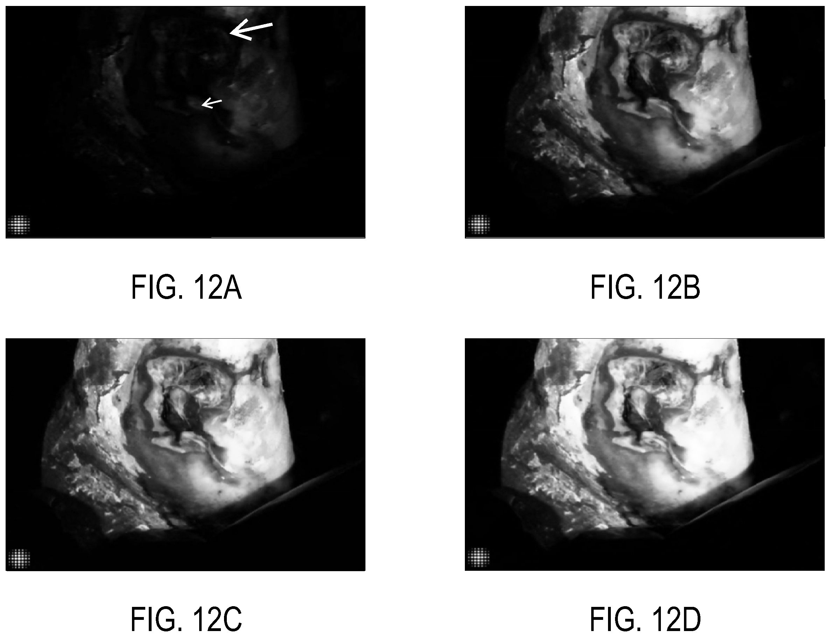

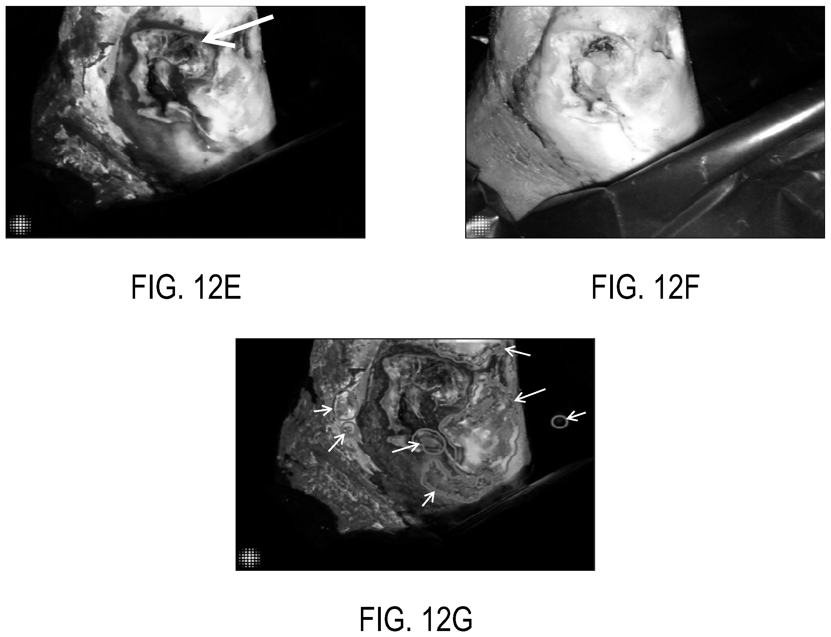

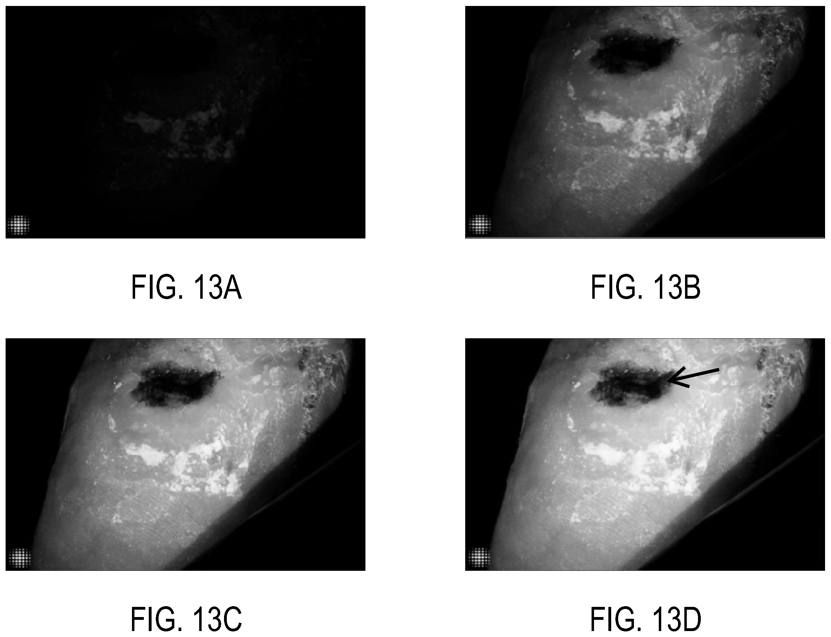

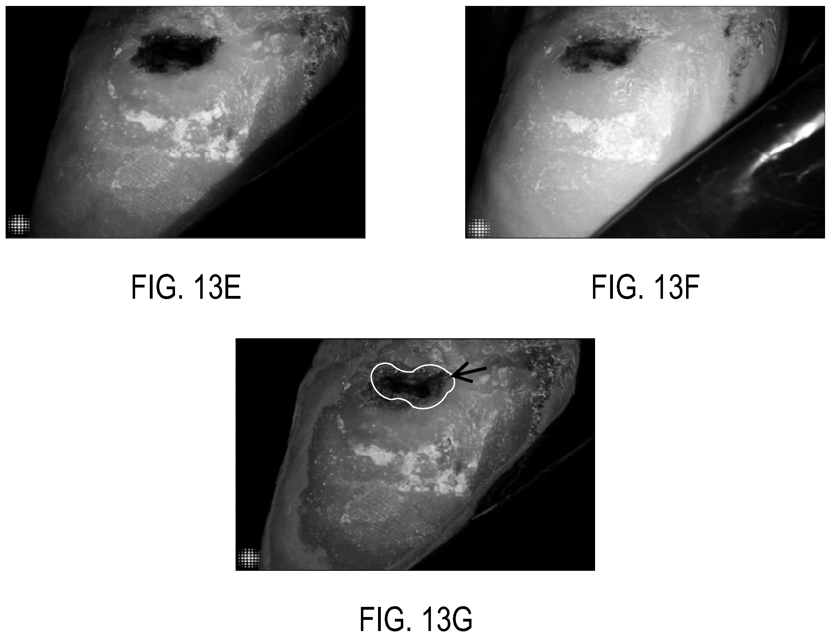

FIGS. 12A-12G and FIGS. 13A-13G illustrate two example sets of images captured using devices and systems in accordance with the present disclosure, and by methods in accordance with the present disclosure. In particular, FIGS. 12A-12E and FIGS. 13A-13E illustrate fluorescence images of respective target areas captured using LED modulation at increasing output power levels of 0.44 mW, 2.3 mW, 4.2 mW, and 6.2 mW, respectively; FIGS. 12E and 13E illustrates fluorescence images taken by a comparative device operating at a nominal output power level of 3.46 mW; FIGS. 12F and 13F illustrate white light images of respective target areas; and FIGS. 12G and 13G illustrate exemplary composite images. It will be understood that, although FIGS. 12A-12G and 13A-13G illustrate the effects of LED modulation, other implementations which rely on exposure period modulation or a combination of LED modulation and exposure period modulation will produce similar effects.

FIGS. 12A-12G correspond to a situation where both the comparative device and the modulated device in accordance with the present disclosure are able to detect the presence of bacteria, but where the modulated device in accordance with the present disclosure is able to detect more areas of contamination and is able to characterize the concentration as "high." As can be seen from FIG. 12A, even at the lowest output power level of 0.44 mW two areas of bacterial contamination may be detected: one near the center of the image (small white arrow) and one near the top-center of the image (large white arrow). By comparison, the area near the top-center of the image (white arrow) appears saturated at the nominal power level as shown in FIG. 12E. In such a situation, the comparative device may not be able to distinguish the bacterial signal from noise such as reflections, etc. Thus, in the composite image of FIG. 12G additional areas of bacterial contamination may be identified and highlighted (see white arrows), as compared to images taken with the nominal device.

FIGS. 13A-13G correspond to a situation where the comparative device is not capable of detecting the presence of bacteria, but where the modulated device in accordance with the present disclosure is able to detect the presence of bacteria and is able to characterize the concentration as "low." As can be seen from FIG. 13D, at the highest output power level of 6.2 mW areas of bacterial fluorescence are detectable along the edges of the wound (see black arrow). By comparison, at lower output power levels (including the nominal power level illustrated in FIG. 13E), the signal from these areas are so weak as to be indistinguishable from background. Thus, in the composite image of FIG. 13G additional areas of bacterial contamination (see black arrow) may be identified and highlighted, as compared to images taken with the nominal device.

The systems, devices, and methods described above thus may provide for fluorescence imaging capable of detecting a wider range of parameters for potential areas of interest *(e.g.,* a wider range of bacterial concentration) and/or may information to accurately, reliably, and/or easily identify potential areas of interest (*e.g.,* bacterial infections) in fluorescence images, thereby improving treatment and reducing errors.

While the above description is presented with regard to fluorescence imaging at a single wavelength of excitation light, in practice the above operations and processes may be performed successively for a plurality of different wavelengths or wavelength bands of excitation light. For example, the above operations and processes may be performed once at a particular wavelength in order to identify and/or classify a first bacterial species, again at a different wavelength in order to identify and/or classify a second bacterial species, and so on.

The exemplary systems, devices, and methods described herein may be performed under the control of a processing system executing computer-readable codes embodied on a non-transitory computer-readable recording medium or communication signals transmitted through a transitory medium. The computer-readable recording medium may be any data storage device that can store data readable by a processing system, and may include both volatile and nonvolatile media, removable and non-removable media, and media readable by a database, a computer, and various other network devices.

Examples of the computer-readable recording medium include, but are not limited to, read-only memory (ROM), random-access memory (RAM), erasable electrically programmable ROM (EEPROM), flash memory or other memory technology, holographic media or other optical disc storage, magnetic storage including magnetic tape and magnetic disk, and solid state storage devices. The computer-readable recording medium may also be distributed over network-coupled computer systems so that the computer-readable code is stored and executed in a distributed fashion. The communication signals transmitted through a transitory medium may include, for example, modulated signals transmitted through wired or wireless transmission paths.

Illustrative examples of systems, methods, and devices described herein are provided below. An embodiment of a system, method, and/or device described herein may include any one or more, and any combination of, the clauses described below:

Clause 1. A portable, hand-held device, comprising: an illumination device including at least one excitation light source and a driver configured to drive the at least one excitation light source to sequentially produce a plurality of output intensities; an imaging device configured to capture a plurality of fluorescence images of a target surface, each one of the plurality of fluorescent images respectively corresponding to one of the plurality of output intensities; a memory; and a processor configured to: co-register the plurality of fluorescence images, divide an image area into a plurality of sections, for each of the plurality of sections, select an image portion from one of the plurality of fluorescence images, and combine the selected image portions to generate a composite image.

Clause 2. The device according to clause 1, wherein the driver is configured to modulate the at least one excitation light source by controlling one or more of a current through the at least one excitation light source, a voltage across the excitation light source, or a duty cycle of a pulse-width modulation signal provided to the excitation light source.

Clause 3. The device according to clause 1 or clause 2, wherein the driver is configured to drive the at least one excitation light source according to a monotonically increasing or monotonically decreasing output waveform having a plurality of steps, wherein an amplitude of each one of the plurality of steps corresponds to a respective one of the plurality of output intensities.

Clause 4. The device according to clause 1 or clause 2, wherein the driver is configured to drive the at least one excitation light source according to an output waveform having a plurality of pulses, wherein an amplitude of each of the pulses corresponds to a respective one of the plurality of output intensities.

Clause 5. The device according to any one of clauses 1 to 4, wherein the at least one excitation light source is configured to output excitation light having a wavelength of 405 nm ± 20 nm.

Clause 6. The device according to any one of clauses 1 to 5, wherein the plurality of output intensities is between four and six output intensities, inclusive.

Clause 7. The device according to any one of clauses 1 to 6, wherein the processor is further configured to quantitatively estimate a concentration of a component of the target surface.

Clause 8. The device according to any one of clauses 1 to 7, wherein the processor is configured to select the image portion for a given one of the plurality of sections by: for each of the plurality of fluorescence images, determining a respective fluorescence signal corresponding to a signature of a component of the target surface; and selecting, as the selected image portion, the corresponding one of the plurality of fluorescence images for which the respective fluorescence signal is highest.

Clause 9. The device according to clause 7 or clause 8, wherein the component of the target surface is a bacterial species.

Clause 10. The device according to clause 9, wherein the bacterial species is at least one of *Pseudomonas aeruginosa, Escherichia coli, Proteus mirabilis, Proteus vulgaris, Enterobacter cloacae, Serratia marcescens, Acinetobacter baumannii, Klebsiella pneumoniae, Klebsiella oxytoca, Morganella morganii, Stenotrophomonas maltophilia, Citrobacter koseri, Citrobacter freundii, Aeromonas hydrophilia, Alcaligenes faecalis, Pseudomonas putida, Staphylococcus aureus, Staphylococcus epidermis, Staphylococcus lugdunensis, Staphylococcus capitis, Corynebacterium striatum, Bacillus cereus, Listeria monocytogenes, Bacteroides fragilis*, *Clostridium perfringens*, *Peptostreptococcus anaerobius, Propionibacterium acnes,* and/or *Veillonella parvula.*

Clause 11. The device according to any one of clauses 1 to 10, wherein the processor is further configured to generate at least one interface element on the composite image.

Clause 12. The device according to clause 11, wherein the at least one interface element is configured to identify an area of the target surface which meets a predetermined condition.

Clause 13. The device according to clause 12, wherein the predetermined condition is that a bacterial concentration in the area exceeds a predetermined threshold.

Clause 14. The device according to any one of clauses 1 to 13, wherein the at least one interface element includes an overlay to highlight a portion of the composite image.

Clause 15. The device according to any one of clauses 1 to 14, further comprising a display device configured to display the composite image.

Clause 16. The device according to any one of clauses 1 to 15, wherein the memory is configured to store the composite image.

Clause 17. The device according to any one of clauses 1 to 16, wherein the imaging device includes a first image sensor configured to detect wavelengths between 470 nm and 520 nm, inclusive.

Clause 18. The device according to any one of clauses 1 to 17, wherein the imaging device includes a second image sensor configured to detect wavelengths between 600 nm and 660 nm, inclusive.

Clause 19. The device according to any one of clauses 1 to 18, wherein the imaging device is configured to capture the plurality of fluorescence images at a rate of approximately two per second.

Clause 20. The device according to any one of clauses 1 to 19, wherein the composite image includes metadata identifying the selected one of the plurality of fluorescence images for each of the plurality of sections.

Clause 21. The device according to any one of clauses 1 to 20, wherein combining the selected image portions includes smoothing discontinuities between adjacent ones of the plurality of sections.

Clause 22. The device according to any one of clauses 1 to 21, wherein the processor includes a trained machine learning algorithm configured to perform the operation of selecting the image portion and/or the operation of combining the selected image portions.

Clause 23. The device according to any one of clauses 1 to 22, wherein the imaging device is further configured to capture a white light image of the target surface in the absence of an output from the at least one excitation light source.

Clause 24. A system, comprising: a display device; an illumination device including an excitation light source and a driver configured to drive the excitation light source to sequentially produce a plurality of output intensities; an imaging device configured to capture a plurality of fluorescence images of a target surface respectively corresponding to the plurality of output intensities; a housing; and circuitry disposed within the housing, the circuitry including a processor configured to: co-register the plurality of fluorescence images, divide an image area into a plurality of sections, for each of the plurality of sections, select an image portion from one of the plurality of fluorescence images, and combine the selected image portions to generate a composite image output to the display device.

Clause 25. A fluorescence imaging method, comprising: capturing a plurality of fluorescence images of a target surface, including sequentially for a plurality of different values of a drive parameter: driving an excitation light source to produce excitation light at an output intensity corresponding to the drive parameter, and capturing a respective fluorescence image of the target surface, wherein the fluorescence image includes an emission response of the target surface to the excitation light; co-registering the plurality of fluorescence images; dividing an image area into a plurality of sections; for each of the plurality of sections, selecting an image portion from one of the plurality of fluorescence images; and combining the selected image portions to generate a composite image.

Clause 26. A portable, hand-held device, comprising: an illumination device including at least one excitation light source configured to produce light at an output intensity; an imaging device including an image sensor array and a timing controller configured to drive the image sensor array to sequentially capture a plurality of fluorescence images of a target surface respectively corresponding to a plurality of exposure periods; a memory; and a processor configured to: co-register the plurality of fluorescence images, divide an image area into a plurality of sections, for each of the plurality of sections, select an image portion from one of the plurality of fluorescence images, and combine the selected image portions to generate a composite image.

Clause 27. The device according to clause 26, wherein the at least one excitation light source is configured to output excitation light having a wavelength of 405 nm ± 20 nm.

Clause 28. The device according to clause 26 or clause 27, wherein the plurality of exposure periods is between four and six exposure periods, inclusive.

Clause 29. The device according to any one of clauses 26 to 28, wherein the processor is further configured to quantitatively estimate a concentration of a component of the target surface.

Clause 30. The device according to any one of clauses 26 to 29, wherein the processor is configured to select the image portion for a given one of the plurality of sections by: for each of the plurality of fluorescence images, determining a respective fluorescence signal corresponding to a signature of a component of the target surface; and selecting, as the selected image portion, the corresponding one of the plurality of fluorescence images for which the respective fluorescence signal is highest.

Clause 31. The device according to clause 29 or clause 30, wherein the component is a bacterial species.

Clause 32. The device according to clause 31, wherein the bacterial species is at least one of *Pseudomonas aeruginosa, Escherichia coli, Proteus mirabilis, Proteus vulgaris, Enterobacter cloacae, Serratia marcescens, Acinetobacter baumannii, Klebsiella pneumoniae, Klebsiella oxytoca, Morganella morganii, Stenotrophomonas maltophilia, Citrobacter koseri, Citrobacter freundii, Aeromonas hydrophilia, Alcaligenes faecalis, Pseudomonas putida, Staphylococcus aureus, Staphylococcus epidermis, Staphylococcus lugdunensis, Staphylococcus capitis, Corynebacterium striatum, Bacillus cereus, Listeria monocytogenes, Bacteroides fragilis*, *Clostridium perfringens*, *Peptostreptococcus anaerobius, Propionibacterium acnes,* and/or *Veillonella parvula.*

Clause 33. The device according to any one of clauses 26 to 32, wherein the processor is further configured to generate at least one interface element on the composite image.

Clause 34. The device according to clause 33, wherein the at least one interface element is configured to identify an area of the target surface which meets a predetermined condition.

Clause 35. The device according to clause 34, wherein the predetermined condition is that a bacterial concentration in the area exceeds a predetermined threshold.

Clause 36. The device according to any one of clauses 26 to 35, further comprising a display device configured to display the composite image.

Clause 37. The device according to any one of clauses 26 to 36, wherein the memory is configured to store the composite image.

Clause 38. The device according to any one of clauses 26 to 37, wherein the imaging device includes a first image sensor configured to detect wavelengths between 470 nm and 520 nm, inclusive.

Clause 39. The device according to any one of clauses 26 to 38, wherein the imaging device includes a second image sensor configured to detect wavelengths between 600 nm and 660 nm, inclusive.

Clause 40. The device according to any one of clauses 26 to 39, wherein the imaging device is configured to capture the plurality of fluorescence images at a rate of approximately two per second.

Clause 41. The device according to any one of clauses 26 to 40, wherein the composite image includes metadata identifying the selected one of the plurality of fluorescence images for each of the plurality of sections.

Clause 42. The device according to any one of clauses 26 to 41, wherein combining the selected image portions includes smoothing discontinuities between adjacent ones of the plurality of sections.

Clause 43. The device according to any one of clauses 26 to 42, wherein the processor includes a trained machine learning algorithm configured to perform the operation of selecting the image portion and/or the operation of combining the selected image portions.

Clause 44. The device according to any one of clauses 26 to 43, wherein the imaging device is further configured to capture a white light image of the target surface in the absence of an output from the at least one excitation light source.

Clause 45. A system, comprising: a display device; an illumination device including an excitation light source configured to produce light at an output intensity; an imaging device including an image sensor array and a timing controller configured to drive the image sensor array to sequentially capture a plurality of fluorescence images of a target surface respectively corresponding to a plurality of exposure periods; a housing; and circuitry disposed within the housing, the circuitry including a processor configured to: co-register the plurality of fluorescence images, divide an image area into a plurality of sections, for each of the plurality of sections, select an image portion from one of the plurality of fluorescence images, combine the selected image portions to generate a composite image, and output the composite image to the display device.

Clause 46. A fluorescence imaging method, comprising: capturing a plurality of fluorescence images of a target surface, including sequentially for a plurality of different values of an image capture parameter: driving an excitation light source to produce excitation light at an output intensity, setting an exposure period of an image sensor to a value corresponding to the drive parameter, and capturing a respective fluorescence image of the target surface, wherein the fluorescence image includes an emission response of the target surface to the excitation light; co-registering the plurality of fluorescence images; dividing an image area into a plurality of sections; for each of the plurality of sections, selecting an image portion from one of the plurality of fluorescence images; and combining the selected image portions to generate a composite image.

Clause 47. A portable, hand-held device, comprising: an illumination device including at least one excitation light source and a driver configured to drive the at least one excitation light source to sequentially produce at least one output intensity; an imaging device an imaging device including an image sensor array and a timing controller configured to drive the image sensor array to sequentially capture a plurality of fluorescence images of a target surface respectively corresponding to at least one exposure period; a memory; and a processor configured to: co-register the plurality of fluorescence images, divide an image area into a plurality of sections, for each of the plurality of sections, select an image portion from one of the plurality of fluorescence images, and combine the selected image portions to generate a composite image.

Clause 48. An imaging method, comprising: capturing a plurality of images of a target surface, including sequentially for a plurality of different values of a drive parameter: driving an light source to produce light at an output intensity corresponding to the drive parameter, and capturing a respective image of the target surface, wherein the image includes an optical response of the target surface to the light; co-registering the plurality of images; dividing an image area into a plurality of sections; for each of the plurality of sections, selecting an image portion from one of the plurality of images; and combining the selected image portions to generate a composite image.

Clause 49. The method according to clause 48, wherein respective ones of the plurality of images are fluorescence images, white-light images, or thermal images.

Clause 50. A portable, hand-held device, comprising: an illumination device including at least one light source configured to produce light at an output intensity; an imaging device including an image sensor array and a timing controller configured to drive the image sensor array to sequentially capture a plurality of images of a target surface respectively corresponding to a plurality of exposure periods; a memory; and a processor configured to: co-register the plurality of images, divide an image area into a plurality of sections, for each of the plurality of sections, select an image portion from one of the plurality of fluorescence images, and combine the selected image portions to generate a composite image.

Clause 51. The device of any one of clauses 1 to 23 configured for imaging of wound components (optionally collagen, blood and/or vasculature, oxygenation and/or perfusion, bone, adipose tissue, exudate, and bacteria).

Clause 52. The device of clause 51 further configured for identification of characteristics captured in fluorescent images of the wound components.

Clause 53. Use of the device of any one of clauses 1-23 or 51 or 52 for imaging of wound components (optionally collagen, blood and/or vasculature, oxygenation and/or perfusion, bone, adipose tissue, exudate, and bacteria).

Clause 54. The use of clause 53 for identification of characteristics captured in fluorescent images of the wound components.

Clause 55. The device of any one of clauses 1 to 23, wherein the device is configured to capture images and identify characteristics of excised tissue.

Clause 56. The device of clause 55, wherein the excised tissue is cancerous tissue (e.g., lumpectomy for breast cancer surgery).

Clause 57. The device of clause 56 wherein the device is configured to identify characteristics of the excised cancerous tissue, for example, tissue components, tumor size, tumor edge, tumor boundaries, and tissue vascularization.

Clause 58. Use of the device of any one of clauses 1 to 23 or 55 or 56 or 57 to capture images and identify characteristics of excised tissue.

Clause 59. The use of clause 58, wherein the excised tissue is cancerous tissue (e.g., lumpectomy for breast cancer surgery).

Clause 60. The use of clause 59 to identify characteristics of the excised cancerous tissue, for example, tissue components, tumor size, tumor edge, tumor boundaries, and tissue vascularization.

Clause 61. The system of clause 24 configured for imaging of wound components (optionally collagen, blood and/or vasculature, oxygenation and/or perfusion, bone, adipose tissue, exudate, and bacteria).

Clause 62. The system of clause 61 further configured for identification of characteristics captured in fluorescent images of the wound components.

Clause 63. Use of the system of any one of clauses 24 or 61 or 62 for imaging of wound components (optionally collagen, blood and/or vasculature, oxygenation and/or perfusion, bone, adipose tissue, exudate, and bacteria).

Clause 64. The use of clause 63 for identification of characteristics captured in fluorescent images of the wound components.

Clause 65. The system of clause 24, wherein the system is configured to capture images and identify characteristics of excised tissue.

Clause 66. The system of clause 65, wherein the excised tissue is cancerous tissue (e.g., lumpectomy for breast cancer surgery).

Clause 67. The system of clause 66 wherein the system configured to identify characteristics of the excised cancerous tissue, for example, tissue components, tumor size, tumor edge, tumor boundaries, and tissue vascularization.

Clause 68. The system of clause 24 or 61 or 62 or 65 or 66 or 67, wherein the system is a hand-held system.

Clause 69. Use of the system of any one of clauses 24 or 65 to 68 to capture images and identify characteristics of excised tissue.

Clause 70. The use of clause 69, wherein the excised tissue is cancerous tissue (e.g., lumpectomy for breast cancer surgery).

Clause 71. The use of clause 70 to identify characteristics of the excised cancerous tissue, for example, tissue components, tumor size, tumor edge, tumor boundaries, and tissue vascularization.

Clause 72. The method of clause 25, wherein the method uses the device of any one of clauses from 1 to 23 or of any one of clauses from 26 to 44 or of clause 47 or of any one of clauses from 51 to 52 or of any one of clauses from 55 to 57.

Clause 73. The method of clause 25, wherein the method uses the system of any one of clauses 24 or 45 or 61 or 62 of any one of clauses from 65 to 68.

Clause 74. The method of clause 46, wherein the method uses the device of any one of clauses from 1 to 23 or of any one of clauses from 26 to 44 or of clause 47 or of any one of clauses from 51 to 52 or of any one of clauses from 55 to 57.

Clause 75. The method of clause 46, wherein the method uses the system of any one of clauses 24 or 45 or 61 or 62 of any one of clauses from 65 to 68.

Clause 76. The method of any one clauses 25 or 46 or 72 or 73 or 74 or 75, wherein the method is a computer implemented method.

Clause 77. Controller configured for commanding execution of the method of any one clauses 25 or 46 or 72 or 73 or 74 or 75 or 76.

Clause 78. Controller according to clause 77, wherein the controller comprises an electronic processor, optionally one or more of computer processors, microprocessors, digital signal processors, controllers or microcontrollers.

The above description and associated figures teach the best mode of the disclosed devices, systems, and methods, and are intended to be illustrative and not restrictive. Many embodiments and applications other than the examples provided would be apparent to those skilled in the art upon reading the above description. The scope should be determined, not with reference to the above description, but instead with reference to the appended claims, along with the full scope of equivalents to which such claims are entitled. It is anticipated and intended that future developments will occur in the technologies discussed herein, and that the disclosed systems and methods will be incorporated into future embodiments. In sum, it should be understood that the application is capable of modification and variation.

All terms used in the claims are intended to be given their broadest reasonable constructions and their ordinary meanings as understood by those knowledgeable in the technologies described herein unless an explicit indication to the contrary is made herein. In particular, the use of the singular articles such as "a," "the," "said," etc. should be read to recite one or more of the indicated elements unless a claim recites an explicit limitation to the contrary.

The Abstract is provided to allow the reader to quickly ascertain the nature of the technical disclosure. It is submitted with the understanding that it will not be used to interpret or limit the scope or meaning of the claims. In addition, in the foregoing Detailed Description, it can be seen that various features are grouped together in various embodiments for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus, the following claims are hereby incorporated into the Detailed Description, with each claim standing on its own as a separately claimed subject matter.

## Claims

1. A portable, hand-held device, comprising:
an illumination device including at least one excitation light source configured to produce light at an output intensity;
an imaging device including an image sensor array and a timing controller configured to drive the image sensor array to sequentially capture a plurality of fluorescence images of a target surface respectively corresponding to a plurality of exposure periods;
a memory; and
a processor configured to:
co-register the plurality of fluorescence images,
divide an image area into a plurality of sections,
for each of the plurality of sections, select an image portion from one of the plurality of fluorescence images, and
combine the selected image portions to generate a composite image.

2. The device according to claim 1, wherein the at least one excitation light source is configured to output excitation light having a wavelength of 405 nm ± 20 nm.

3. The device according to claim 1 or claim 2, wherein the plurality of exposure periods is between four and six exposure periods, inclusive.

4. The device according to any one of claims 1 to 3, wherein the processor is further configured to quantitatively estimate a concentration of a component of the target surface.

5. The device according to any one of claims 1 to 4, wherein the processor is configured to select the image portion for a given one of the plurality of sections by:
for each of the plurality of fluorescence images, determining a respective fluorescence signal corresponding to a signature of a component of the target surface; and
selecting, as the selected image portion, the corresponding one of the plurality of fluorescence images for which the respective fluorescence signal is highest.

6. The device according to claim 4 or claim 5, wherein the component is a bacterial species, and optionally wherein the bacterial species is at least one of *Pseudomonas aeruginosa, Escherichia coli, Proteus mirabilis, Proteus vulgaris, Enterobacter cloacae, Serratia marcescens, Acinetobacter baumannii, Klebsiella pneumoniae, Klebsiella oxytoca, Morganella morganii, Stenotrophomonas maltophilia, Citrobacter koseri, Citrobacter freundii, Aeromonas hydrophilia, Alcaligenes faecalis, Pseudomonas putida, Staphylococcus aureus, Staphylococcus epidermis, Staphylococcus lugdunensis, Staphylococcus capitis, Corynebacterium striatum, Bacillus cereus, Listeria monocytogenes, Bacteroides fragilis*, *Clostridium perfringens*, *Peptostreptococcus anaerobius, Propionibacterium acnes,* and/or *Veillonella parvula.*

7. The device according to any one of claims 1 to 6, wherein the processor is further configured to generate at least one interface element on the composite image.

8. The device according to claim 7, wherein the at least one interface element is configured to identify an area of the target surface which meets a predetermined condition, and
optionally wherein the predetermined condition is that a bacterial concentration in the area exceeds a predetermined threshold.

9. The device according to any one of claims 1 to 8, further comprising a display device configured to display the composite image.

10. The device according to any one of claims 1 to 9, wherein the imaging device includes:
a first image sensor configured to detect wavelengths between 470 nm and 520 nm, inclusive, and/or
a second image sensor configured to detect wavelengths between 600 nm and 660 nm, inclusive.

11. The device according to any one of claims 1 to 10, wherein the imaging device is configured to capture the plurality of fluorescence images at a rate of approximately two per second, and/or wherein the imaging device is further configured to capture a white light image of the target surface in the absence of an output from the at least one excitation light source.

12. The device according to any one of claims 1 to 11, wherein the composite image includes metadata identifying the selected one of the plurality of fluorescence images for each of the plurality of sections.

13. The device according to any one of claims 1 to 12, wherein combining the selected image portions includes smoothing discontinuities between adjacent ones of the plurality of sections.

14. The device according to any one of claims 1 to 13, wherein the processor includes a trained machine learning algorithm configured to perform the operation of selecting the image portion and/or the operation of combining the selected image portions.

15. A system, comprising:
a display device;
an illumination device including an excitation light source configured to produce light at an output intensity;
an imaging device including an image sensor array and a timing controller configured to drive the image sensor array to sequentially capture a plurality of fluorescence images of a target surface respectively corresponding to a plurality of exposure periods;
a housing; and
circuitry disposed within the housing, the circuitry including a processor configured to:
co-register the plurality of fluorescence images,
divide an image area into a plurality of sections,
for each of the plurality of sections, select an image portion from one of the plurality of fluorescence images,
combine the selected image portions to generate a composite image, and
output the composite image to the display device.
